(19) Europäisches Patentamt
European Patent Office
Office européen des brevets

(11) EP 4 140 480 A1

(12) # EUROPEAN PATENT APPLICATION
published in accordance with Art. 153(4) EPC

(43) Date of publication:
**01.03.2023 Bulletin 2023/09**

(21) Application number: **21793182.3**

(22) Date of filing: **22.04.2021**

(51) International Patent Classification (IPC):
***A61K 31/375*** *(2006.01)* ***A61K 31/355*** *(2006.01)*
***A61P 35/00*** *(2006.01)* ***A61K 31/44*** *(2006.01)*

(52) Cooperative Patent Classification (CPC):
**A61K 31/355; A61K 31/375; A61K 31/44; A61P 35/00**

(86) International application number:
**PCT/CN2021/088840**

(87) International publication number:
**WO 2021/213455 (28.10.2021 Gazette 2021/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.04.2020 CN 202010329182**

(71) Applicant: **NATURAL MEDICINE INSTITUTE OF ZHEJIANG YANGSHENGTANG CO., LTD.**
**Xihu District**
**Hangzhou**
**Zhejiang 310024 (CN)**

(72) Inventors:
- **XU, Nuo**
  **Hangzhou, Zhejiang 310024 (CN)**
- **LIN, Jian**
  **Beijing 100871 (CN)**

(74) Representative: **Böhm, Brigitte**
**Weickmann & Weickmann**
**Patent- und Rechtsanwälte PartmbB**
**Postfach 860 820**
**81635 München (DE)**

(54) ## DRUG COMBINATION AND USE THEREOF

(57) Provided is a drug combination for prevention and/or treatment of tumors. The drug combination includes: a) an ascorbic acid or a derivative thereof in an amount effective for prevention and/or treatment, and b) a selective PDE4 inhibitor in an amount effective for prevention and/or treatment.

EP 4 140 480 A1

## Description

### TECHNICAL FIELD

The present application relates to the field of biological medicine, and in particular, relates to a pharmaceutical combination and use thereof.

### BACKGROUND OF THE INVENTION

Malignant tumors are diseases that are seriously harmful to human health at present. Global cancer statistics in 2018 showed that there were 18.19 million new cancer cases and 9.6 million cancer deaths across the world. Take colorectal cancer as an example, the colorectal cancer accounts for 10.2% of the total number of cancer cases and 9.2% of the number of deaths. That is, there are about 1.85 million new cases of colorectal cancer and 880,000 death cases each year. In China, there are about 370,000 new cases of colorectal cancer every year. In the clinical therapy for colorectal cancer, oxaliplatin is mainly used, with the clinical chemotherapy regimens including oxaliplatin + xeloda, and oxaliplatin + fluorouracil. However, these chemotherapy regimens have serious side effects, for example, damaging the digestive system and the blood system, and drug resistance often occurs. In addition, more than 30% of patients with colon cancer in clinical practice are insensitive to platinum chemotherapeutics. All these indicate that, in addition to anti-tumor effects, the chemotherapeutics also exhibit toxic and side effects or drug-resistant defects to different extents. In particular, there are some tumor patients who are insensitive to the chemotherapeutics. Therefore, it is an urgent problem for the medical community all over the world to find more effective anti-tumor medicaments and methods.

### SUMMARY OF THE INVENTION

The present application provides a pharmaceutical combination, and a use or administration method thereof, and mainly involves taking a selective PDE4 inhibitor and an ascorbic acid or a derivative thereof as a pharmaceutical combination for treating a tumor, or administering the selective PDE4 inhibitor and the ascorbic acid or the derivative thereof in combination to treat a tumor. In addition, the selective PDE4 inhibitor, the ascorbic acid or the derivative thereof, or a platinum compound can be taken as a pharmaceutical combination for treating a tumor; or the selective PDE4 inhibitor, the ascorbic acid or the derivative thereof, or the platinum compound can be administered in combination to treat a tumor. The present application has at least one of the following technical effects:

1) the pharmaceutical combination consisting of the selective PDE4 inhibitor and the ascorbic acid or the derivative thereof can be used as an alternative therapy to the traditional chemotherapeutics, in order to avoid toxic and side effects caused by the traditional chemotherapy on the human body;
2) the pharmaceutical combination consisting of the selective PDE4 inhibitor and the ascorbic acid or the derivative thereof is for use in the treatment of tumor patients who are insensitive to traditional chemotherapeutics or undergo drug resistance, in order to achieve a therapeutic effect;
3) the pharmaceutical combination consisting of the selective PDE4 inhibitor and the ascorbic acid or the derivative thereof can be combined with traditional chemotherapeutics (for example, platinum compounds) to reduce the dose of chemotherapeutics, thereby reducing the toxic and side effects of the traditional chemotherapeutics;
4) the combination of the selective PDE4 inhibitor and the ascorbic acid or the derivative thereof has achieved an unexpected inhibitory effect on tumors and significantly reduces the administration dose of a single medicament having an equivalent effect, which improves the safety of medication; and
5) the pharmaceutical combination specifically inhibits the growth of tumors or tumor cells, and has no effect on other physiological indicators (for example, body weight) of a subject, exhibiting the pharmaceutical safety.

The present application provides a pharmaceutical combination, comprising:

a) a prophylactically and/or therapeutically effective amount of ascorbic acid or a derivative thereof; and
b) a prophylactically and/or therapeutically effective amount of a selective PDE4 inhibitor.

In some embodiments, the pharmaceutical combination further comprises a prophylactically and/or therapeutically effective amount of platinum compound.
In some embodiments, said platinum compound comprises oxaliplatin, cisplatin and/or carboplatin.
In some embodiments, said selective PDE4 inhibitor comprises apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

In some embodiments, said selective PDE4 inhibitor comprises a compound represented by Formula I or a pharmaceutically acceptable salt thereof,

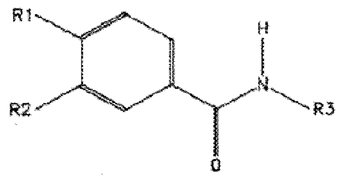 (I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and
R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,
R32 is hydrogen, hydroxy, halogen, amino, trifluoromethyl, $C_1$-$C_4$ alkyl, or $C_1$-$C_4$ alkoxy,
R33 is hydrogen, halogen, $C_1$-$C_4$ alkyl, or $C_1$-$C_4$ alkoxy,
R34 is hydroxy, halogen, cyano, carboxy, $C_1$-$C_4$ alkyl, $C_1$-$C_4$ alkoxy, $C_1$-$C_4$ alkoxycarbonyl, or amino,
R35 is hydrogen, halogen, amino, or $C_1$-$C_4$ alkyl,
R36 is hydrogen or halogen, and
R37 is hydrogen or halogen.

In some embodiments, said selective PDE4 inhibitor comprises roflumilast or a derivative thereof.
In some embodiments, the derivative of said roflumilast comprises benzanilide and/or 4-methylbenzanilide.
In some embodiments, the derivative of said ascorbic acid comprises dehydroascorbic acid and/or pharmaceutically acceptable ascorbate.
In some embodiments, said pharmaceutically acceptable ascorbate comprises sodium ascorbate.
In some embodiments, an effective amount ratio of said selective PDE4 inhibitor to said ascorbic acid or the derivative thereof is about 1:50 to about 1:500.
In some embodiments, a mass ratio of said selective PDE4 inhibitor to said ascorbic acid or the derivative thereof is about 1:50 to about 1:500.
In some embodiments, an effective amount ratio of said selective PDE4 inhibitor to said ascorbic acid or the derivative thereof to said platinum compound is about 1:50:1 to about 1:500:1.
In some embodiments, said selective PDE4 inhibitor and said ascorbic acid or the derivative thereof are each present in a different container, respectively.
In some embodiments, said selective PDE4 inhibitor, said ascorbic acid or the derivative thereof, and said platinum compound are each present in a different container, respectively.
In some embodiments, said pharmaceutical combination comprises a first formulation and a second formulation, and said first formulation comprises said ascorbic acid or the derivative thereof and a first pharmaceutically acceptable carrier, and said second formulation comprises said selective PDE4 inhibitor and a second pharmaceutically acceptable carrier.
In some embodiments, said pharmaceutical combination comprises a first formulation, a second formulation, and a third formulation, said first formulation comprises said ascorbic acid or the derivative thereof and a first pharmaceutically acceptable carrier, said second formulation comprises said selective PDE4 inhibitor and a second pharmaceutically acceptable carrier, and said third formulation comprises said platinum compound and a third pharmaceutically acceptable carrier.
In some embodiments, said pharmaceutical combination comprises a pharmaceutical composition, which comprises said selective PDE4 inhibitor and said ascorbic acid or the derivative thereof.
In some embodiments, said pharmaceutical composition further comprises a platinum compound.
In some embodiments, said selective PDE4 inhibitor in said pharmaceutical composition has a content of about 1% to about 5% (w/w).
In some embodiments, said ascorbic acid or the derivative thereof in said pharmaceutical composition has a content of about 90% to about 98% (w/w).
In some embodiments, said platinum compound in said pharmaceutical composition has a content of about 1% to about 5% (w/w).
In another aspect, the present application further provides a kit, comprising the pharmaceutical combination

defined in the present application.

In some embodiments, the pharmaceutical combination or kit is for use in the prevention and/or treatment of a tumor.

In some embodiments, said tumor comprises a solid tumor and/or a non-solid tumor.

In some embodiments, said solid tumor comprises a colon cancer and/or melanoma.

In some embodiments, said tumor comprises a tumor or tumor cell having no response to the platinum compound.

In another aspect, the present application further provides use of a combination of ascorbic acid or a derivative thereof and a selective PDE4 inhibitor in the preparation of a medicament for preventing and/or treating a tumor.

In some embodiments, the present application further provides use of a combination of ascorbic acid or a derivative thereof, a selective PDE4 inhibitor, and a platinum compound in the preparation of a medicament for preventing and/or treating a tumor.

In some embodiments, said platinum compound comprises oxaliplatin, cisplatin and/or carboplatin.

In some embodiments, said selective PDE4 inhibitor comprises apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

In some embodiments, said selective PDE4 inhibitor comprises a compound represented by Formula I or a pharmaceutically acceptable salt thereof,

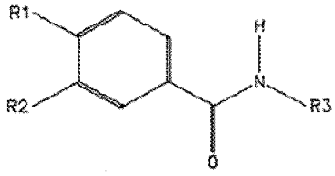

(I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is C1-C4 alkoxy fully or partially substituted by fluorine, and

R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,
R32 is hydrogen, hydroxy, halogen, amino, trifluoromethyl, $C_1$-$C_4$ alkyl, or $C_1$-$C_4$ alkoxy,
R33 is hydrogen, halogen, $C_1$-$C_4$ alkyl, or $C_1$-$C_4$ alkoxy,
R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl,
R36 is hydrogen or halogen, and
R37 is hydrogen or halogen.

In some embodiments, said selective PDE4 inhibitor comprises roflumilast or a derivative thereof.

In some embodiments, the derivative of said roflumilast comprises benzanilide and/or 4-methylbenzanilide.

In some embodiments, the derivative of said ascorbic acid comprises dehydroascorbic acid and/or pharmaceutically acceptable ascorbate.

In some embodiments, said pharmaceutically acceptable ascorbate comprises sodium ascorbate.

In some embodiments, said tumor comprises a solid tumor and/or a non-solid tumor.

In some embodiments, said solid tumor comprises a colon cancer and/or melanoma.

In some embodiments, said tumor comprises a tumor or tumor cell having no response to said platinum compound.

In some embodiments, said selective PDE4 inhibitor and said ascorbic acid or the derivative thereof are formulated to be simultaneously administered to a subject.

In some embodiments, said selective PDE4 inhibitor and said ascorbic acid or the derivative thereof are formulated to be separately administered to a subject.

In some embodiments, said selective PDE4 inhibitor, said ascorbic acid or the derivative thereof, and said platinum compound are formulated to be simultaneously administered to a subject.

In some embodiments, said selective PDE4 inhibitor, said ascorbic acid or the derivative thereof, and said platinum compound are separately formulated to be administered to a subject.

In some embodiments, said medicament is formulated such that said selective PDE4 inhibitor is administered

at a dose of about 1 mg/kg to about 10 mg/kg.

In some embodiments, said medicament is formulated such that said ascorbic acid or the derivative thereof is administered at a dose of about 0.5 g/kg to about 2 g/kg.

In some embodiments, said medicament is formulated such that said platinum compound is administered at a dose of about 1 mg/kg to about 10 mg/kg.

In some embodiments, said medicament is formulated such that said selective PDE4 inhibitor and said ascorbic acid or the derivative thereof are administered at a mass ratio of 1:50 to about 1:500.

In some embodiments, said medicament is formulated such that said selective PDE4 inhibitor and said ascorbic acid

or the derivative thereof are administered at an effective amount ratio of about 1:50 to about 1:500.

In some embodiments, said medicament is formulated such that said selective PDE4 inhibitor, said ascorbic acid or the derivative thereof, and said platinum compound are administered at a mass ratio of about 1:50:1 to about 1:500:1.

In some embodiments, said medicament is formulated such that said selective PDE4 inhibitor and said ascorbic acid or the derivative thereof are administered at an effective amount ratio of about 1:50 to about 1:500.

In some embodiments, said medicament is formulated such that said selective PDE4 inhibitor, said ascorbic acid or the derivative thereof, and said platinum compound are administered at an effective amount ratio of about 1:50:1 to about 1:500:1.

In another aspect, the present application further provides a method for preventing and/or treating a tumor, comprising administering:

a) an effective amount of ascorbic acid or a derivative thereof; and
b) an effective amount of a selective PDE4 inhibitor, to a subject in need thereof.

In some embodiments, the method further comprises administering an effective amount of platinum compound to a subject in need thereof.

In some embodiments, said platinum compound comprises oxaliplatin, cisplatin and/or carboplatin.

In some embodiments, said selective PDE4 inhibitor comprises apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

In some embodiments, said selective PDE4 inhibitor comprises a compound represented by Formula I or a pharmaceutically acceptable salt thereof,

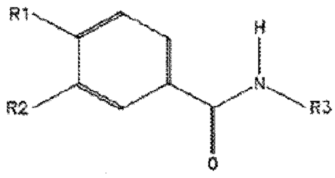 (I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and

R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,
R32 is hydrogen, hydroxy, halogen, amino, trifluoromethyl, $C_1$-$C_4$ alkyl, or $C_1$-$C_4$ alkoxy,
R33 is hydrogen, halogen, $C_1$-$C_4$ alkyl, or $C_1$-$C_4$ alkoxy,
R34 is hydroxy, halogen, cyano, carboxy, $C_1$-$C_4$ alkyl, $C_1$-$C_4$ alkoxy, $C_1$-$C_4$ alkoxycarbonyl, or amino,
R35 is hydrogen, halogen, amino, or $C_1$-$C_4$ alkyl,
R36 is hydrogen or halogen, and
R37 is hydrogen or halogen.

In some embodiments, said selective PDE4 inhibitor comprises roflumilast or a derivative thereof.

In some embodiments, the derivative of said roflumilast comprises benzanilide and/or 4-methylbenzanilide.

In some embodiments, the derivative of said ascorbic acid comprises dehydroascorbic acid and/or pharmaceutically acceptable ascorbate.

In some embodiments, said pharmaceutically acceptable ascorbate comprises sodium ascorbate.

In some embodiments, said subject is a tumor patient having no response to said platinum compound.

In some embodiments, said tumor comprises a solid tumor and/or a non-solid tumor.

In some embodiments, said solid tumor comprises a colon cancer and/or melanoma.

In some embodiments, said tumor comprises a tumor or tumor cell having no response to said platinum compound.

In some embodiments, said selective PDE4 inhibitor and said ascorbic acid or the derivative thereof are simultaneously administered to said subject.

In some embodiments, said selective PDE4 inhibitor and said ascorbic acid or the derivative thereof are sequentially administered to said subject.

In some embodiments, said selective PDE4 inhibitor, said ascorbic acid or the derivative thereof, and said platinum compound are simultaneously administered to said subject.

In some embodiments, said selective PDE4 inhibitor, said ascorbic acid or the derivative thereof, and said platinum compound are sequentially administered to said subject.

In some embodiments, said selective PDE4 inhibitor is administered at a dose of about 1 mg/kg to about 10 mg/kg.

In some embodiments, said ascorbic acid or the derivative thereof is administered at a dose of about 0.5 g/kg to about 2 g/kg.

In some embodiments, said platinum compound is administered at a dose of about 1 mg/kg to about 10 mg/kg.

In some embodiments, said selective PDE4 inhibitor and said ascorbic acid or the derivative thereof are administered at a mass ratio of about 1:50 to about 1:500.

In some embodiments, said selective PDE4 inhibitor and said ascorbic acid or the derivative thereof are administered at an effective amount ratio of about 1:50 to about 1:500.

In some embodiments, said selective PDE4 inhibitor, said ascorbic acid or the derivative thereof, and said platinum compound are administered at a mass ratio of about 1:50:1 to about 1:500:1.

In some embodiments, said selective PDE4 inhibitor, said ascorbic acid or the derivative thereof, and said platinum compound are administered at an effective amount ratio of about 1:50:1 to about 1:500:1.

Other aspects and advantages of the present application can be readily perceived by those skilled in the art from the detailed description below. The detailed description below only shows and describes the exemplary embodiments of the present application. As would be appreciated by those skilled in the art, the content of the present application allows those killed in the art to change the specific embodiments disclosed without departing from the spirit and scope involved in the present application. Accordingly, the accompanying drawings and the description in the specification of the present application are merely for an exemplary but not restrictive purpose.

## BRIEF DESCRIPTION OF THE DRAWING

The specific features of the invention involved in the present application are as set forth in the appended claims. The features and advantages of the invention involved in the present application can be better understood by referring to the exemplary embodiments and the accompanying drawings described in detail below. A brief description of the drawings is as follows:

FIG. 1 shows the results of luciferin fluorescence imaging of mouse colon cancer treated with roflumilast and sodium ascorbate in combination according to the present application;

FIG. 2 shows the quantitative statistical results of luciferin fluorescence imaging of mouse colon cancer treated with roflumilast and sodium ascorbate in combination according to the present application;

FIG. 3 shows the statistical results on the volume changes of mouse colon cancer treated with roflumilast and sodium ascorbate in combination according to the present application;

FIG. 4 shows the effects of roflumilast and sodium ascorbate on the body weight of mice according to the present application;

FIG. 5 shows the statistical results on the volume changes of mouse colon cancer treated with roflumilast, sodium ascorbate, and oxaliplatin according to the present application; and

FIG. 6 shows the effects of roflumilast, sodium ascorbate, and oxaliplatin on the body weight of mice according to the present application.

## DETAILED DESCRIPTION

## DEFINITION OF TERM

In the present application, the term "having no response to a platinum compound" generally refers to the situation that, in the anti-tumor field, the traditional chemotherapies using platinum compounds cannot achieve an expected anti-tumor effect, and the platinum compounds cannot inhibit the proliferation of tumor cells. This situation may comprise examples where the platinum compounds cannot achieve the anti-tumor effect all the way, and may also include examples where the resistance to the platinum compounds occurs during treatment as the treatment progresses. The traditional chemotherapies using the platinum compounds may be known or readily known to those skilled in the art. The platinum compounds may comprise cisplatin, carboplatin, oxaliplatin, nedaplatin, etc.

In the present application, the term "administering ... to the subject sequentially" generally refers to the situation that, when the active ingredients of the pharmaceutical combination are administered to the subject, the time interval between the time when the first active ingredient is administered and the time when the last active ingredient is administered is greater than 1 hour. For example, the active ingredients of the pharmaceutical combination may comprise the selective PDE4 inhibitor, the ascorbic acid and the derivative thereof, and the platinum compound according to the present application.

In the present application, the term "administering ... to the subject simultaneously" generally refers to the situation that, when the active ingredients of the pharmaceutical combination are simultaneously administered to the subject in the form of a composition/mixture, or when the active ingredients of the pharmaceutical combination are administered to the subject, the time interval between the time when the first active ingredient is administered and the time when the last active ingredient is administered is not greater than 1 hour. For example, the active ingredients of the pharmaceutical combination may comprise the selective PDE4 inhibitor, the ascorbic acid and the derivative thereof, and the platinum compound according to the present application.

In the present application, the term "PDE4" generally refers to a member of the PDE4 subfamily in the phosphodiesterases (PDEs) family, or a functional variant thereof, which is capable of destroying a phosphodiester bond. PDEs have the function of hydrolyzing the second messengers (cAMP, cyclic adenosine monophosphate or cGMP, cyclic guanosine monophosphate) in the cells, and can degrade the cAMP or cGMP in the cells to end the biochemical action mediated by these second messengers. PDEs are a large family of multiple genes. In mammals, they can be divided into 12 families, namely PDE1-PDE12, in terms of 1) amino acid sequence, 2) substrate specificity, 3) regulatory properties, 4) pharmacological properties, and 5) tissue distribution. In the PDE nomenclature, the Arabic numeral is used to indicate the PDE family, followed by a capital letter indicating a gene in the family, and then followed by a second Arabic numeral indicating a splice variant derived from a single gene (for example, PDE1C3: family 1, gene C, splice variant 3). The amino acid sequences of different PDEs in the same family may show great differences, but are functionally relevant. PDEs have different substrate specificities. For example, PDE4 is a selective cAMP hydrolase, and in humans, it can be divided into 4 subtypes: PDE4A, 4B, 4C and 4D.

In the present application, the term "selective PDE4 inhibitor" generally refers to an inhibitor that selectively acts on the PDE4 subfamily in the PDE family. Among all members of the PDE family, it mainly shows an inhibitory effect on PDE4, but substantially has no inhibitory effect on other PDE family members except for PDE4. In other words, the selective PDE4 inhibitor is mainly with respect to other members of the PDE family, and the possibility of its inhibitory effect on other molecules outside the PDE family is not ruled out. The selective PDE4 inhibitor may show the inhibitory effect on one or more subtypes in the PDE4 family.

In the present application, the term "pharmaceutical combination" generally refers to a combination comprising at least two active ingredients/therapeutic agents. In some embodiments, each active ingredient/therapeutic agent may be prepared as an independent formulation (solid, liquid, gel, etc.) ; in some embodiments, each active ingredient/therapeutic agent may be present in a different container, and when needed, may also be formulated into a desired formulation with a suitable carrier simultaneously or respectively; in some embodiments, each active ingredient/therapeutic agent may be from different sources (for example, prepared, manufactured or sold by different merchants) ; and in some embodiments, each active ingredient/therapeutic agent may form a pharmaceutical composition in a mixed form.

As used herein, the term "pharmaceutical composition" generally refers to a mixture, which comprises at least one active ingredient to be administered to a subject to treat a specific disease or condition affecting the individual. It allows the active ingredient to remain in an effective form and contains no additional component that has unacceptable toxicity to the subject to which the composition is to be administered. This composition may be sterile, or may contain a pharmaceutically acceptable carrier.

As used herein, the term "inhibitor" generally refers to a compound/substance or composition that is capable of completely or partially preventing or reducing the physiological function of one or more specific biomolecules (for example, proteins, polypeptides, lipopolysaccharides, glycoproteins, ribonucleoprotein complexes, etc.). Reducing the physiological function of one or more specific proteins may involve a reduction in the activity (for example, the capability

of binding to other molecules) of the protein itself or in the amount of the protein itself. In certain embodiments, the inhibitor may exist as different crystals, amorphous substances, pharmaceutically acceptable salts, hydrates, and solvates. In some embodiments, the inhibitor can block the activation of cell signaling pathways.

In the present application, the term "functional variant" generally refers to that a functional variant may comprise a molecule that undergoes an amino acid modification (for example, group substitution, etc.) or the insertion, substitution, and/or deletion of one or more amino acids on an original protein sequence, the function of which is however retained. For example, the functional variant may have a biological activity (function) better than that of the original sequence. For example, the retainment is not necessarily complete retainment. For example, the functional variant may substantially retain the function of the original sequence, for example, by at least 50%, 60%, 65%, 70%, 75%, 80%, 85%, 90%, 95%, 96%, 97%, 98%, or 99%. For example, the amino acid sequence of the functional variant may be at least 50%, 70%, 75%, 80%, 85%, 90%, 95%, 96%, 97%, 98%, or 99% identical to the original amino acid sequence.

As used herein, the term "effective amount" or "effective dose" generally refers to an amount sufficient to achieve or at least partially achieve the desired effect. The "therapeutically effective amount" or "therapeutically effective dose" of a drug or therapeutic agent generally means any drug dose that promotes the regression of a disease (this is demonstrated by means of reduced severity of disease symptoms, increased frequency and duration of the asymptomatic period of the disease, or prevention of injury or disability caused by the disease) when used alone or in combination with another therapeutic agent. The "prophylactically effective amount" or "prophylactically effective dose" of a drug generally refers to a drug dose that inhibits the development or recurrence of a disease when administered alone or in combination with another therapeutic agent to a subject at risk of disease progression or recurrence. A variety of methods known to a person skilled in the art may be used to evaluate the ability of therapeutic or prophylactic agent to promote disease regression or inhibit disease progression or recurrence, for instance, in a human subject undergoing a clinical trial, in an animal model system for predicting the efficacy on humans, or in an in vitro assay for measuring the activity of a agent.

In the present application, the term "derivative of ascorbic acid" generally refers to a compound that releases ascorbic acid (vitamin C) in vivo or in vitro, and a solvate, hydrate or salt thereof. This term also comprises ascorbic acid analogs, in which one or more hydroxyl groups of ascorbic acid are substituted by another structural moiety, and the ascorbic acid analogs substantially maintain the stable activity of the ascorbic acid in vitro or in vivo.

In the present application, the term "container" generally refers to any vessel or device suitable for holding medicaments, for example, a medicine box, a medicine bottle, a medicine bag, a blister, a tube, a syringe, etc.

As used herein, the term "administration" generally refers to the introduction of the pharmaceutical combination into the subject's body by any route of introduction or delivery. Any method known to a person skilled in the art for contacting a cell, an organ or a tissue with the pharmaceutical combination may be used, including but not limited to intra-arterial, intranasal, intra-abdominal, intravenous, intramuscular, subcutaneous transdermal or oral administration. A daily dose may be divided into one, two or more doses of a suitable form for administration at one, two or more times during a certain period of time.

As used herein, the term "subject" generally refers to a human or non-human animal, comprising but not limited to a cat, a dog, a horse, a pig, a cow, a goat, a rabbit, a mouse, a rat, or a monkey.

In the present application, the term "carrier" generally refers to any other substance except for the active ingredient, for example, pharmaceutically acceptable substances, compositions or vehicles involved in carrying or transporting a chemical agent, for example, a buffer, a surfactant, a stabilizer, a preservative, an absorption enhancer for enhancing bioavailability, a liquid or solid filler, a diluent, an excipient, a solvent, an encapsulating material and/or other conventional solubilizer or dispersants, etc. Each carrier must be "acceptable" in the sense of being compatible with other ingredients of the formulation, and will not cause harm to the patient. For example, in the present application, a suitable carrier is selected according to the existence form (composition or each as an individual formulation) of each active ingredient (for example, the ascorbic acid, the selective PDE4 inhibitor or the platinum chemotherapeutics). For example, when different active ingredients exist independently, they may coordinate with a suitable carrier (i.e., the first carrier, second carrier, or third carrier of the present application) respectively to form a formulation suitable for administration.

In the present application, the term "formulation", also referred to as a pharmaceutical formulation, generally refers to a medicament which is formulated according to certain dosage form requirements in order to meet the needs of treatment or prevention, and which can be provided to a subject for use. For example, the formulation may contain an active ingredient and a carrier.

In the present application, the term "prevention" generally refers to a prophylactic administration combination for a healthy subject in order to prevent the occurrence of a certain disease or condition. It may also include a prophylactic administration combination for a patient in the early stage of an allergic disease to be treated. "Prevention" does not require the possibility of 100% elimination of the occurrence of a disease or condition. In other words, "prevention" generally means reducing the probability or degree of occurrence of a disease or condition in the presence of the administration combination.

In the present application, the term "treatment" generally refers to a clinical intervention used to change a natural course in a treated individual or cell in a clinical pathological process. It may include improving the state of a disease, eliminating a lesion, or improving prognosis.

In the present application, the term "tumor" generally refers to a neoplasm or solid lesion resulting from abnormal cell growth. In the present application, the tumor may be a solid tumor or a non-solid tumor. In some embodiments, a tangible mass that can be touched by means of clinical examinations such as X-ray radiography, CT scan, B-ultrasonography, or palpation can be called a solid tumor; and a tumor (for example, leukemia) that cannot be seen or touched by means of X-ray radiography, CT scan, B-ultrasonography, and palpation can be called a non-solid tumor.

In the present application, the term "alkoxy" generally refers to an -OR group, with R that may be an optionally substituted alkyl.

In the present application, the term "alkyl" generally refers to straight-chain and branched-chain saturated alkyl. Non-limiting examples of alkyl may comprise methyl, ethyl, and straight-chain and branched-chain propyl and butyl.

In the present application, the term "halogen" refers to halogens of group VIIA in the periodic table, for example, F, Cl, Br, and I.

In the present application, the term "amino" generally refers to an -NH group, in which one or two of the hydrogen atoms may optionally be substituted, for example, with alkyl, substituted alkyl, cycloalkyl, aryl, or heteroaryl.

In the present application, the term "comprise" or "include" generally refers to the inclusion of explicitly specified features, but not excluding other elements.

In the present application, the term "about" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, and 10% above or below a specified value.

In the present application, as known to those skilled in the art, terms such as "alkyl", "alkenyl", and "cycloalkyl" can be added with an identifier in front of the name to indicate the number of atoms present in the group in a specific situation, for example, $C_1$-$C_4$ alkyl, $C_3$-$C_7$ cycloalkoxy, $C_1$-$C_4$ alkylcarbonylamino, etc., and the subscript number following "C" indicates the number of carbon atoms present in the group. For example, $C_3$ alkyl refers to an alkyl group having three carbon atoms (for example, n-propyl, isopropyl); and in $C_{1-10}$, the members of the group may have any number of carbon atoms falling within the range of 1-4 atom.

## DETAILED DESCRIPTION OF THE INVENTION

In one aspect, the present application provides a pharmaceutical combination, comprising:

a) a prophylactically and/or therapeutically effective amount of ascorbic acid or a derivative thereof; and
b) a prophylactically and/or therapeutically effective amount of a selective PDE4 inhibitor.

For example, the pharmaceutical combination may further comprise a prophylactically and/or therapeutically effective amount of platinum compound.

In another aspect, the present application further provides use of a combination of ascorbic acid or a derivative thereof and a selective PDE4 inhibitor in the preparation of a medicament for preventing and/or treating a tumor. The present application further provides use of a combination of ascorbic acid or a derivative thereof, a selective PDE4 inhibitor, and the platinum compound in the preparation of a medicament for preventing and/or treating a tumor.

In another aspect, the present application further provides a method for preventing and/or treating a tumor, comprising administering:

a) an effective amount of ascorbic acid or a derivative thereof; and
b) an effective amount of a selective PDE4 inhibitor, to a subject in need thereof.

In some embodiments, the method further comprises administering an effective amount of platinum compound to a subject in need thereof.

In another aspect, the present application further provides a kit, which may comprise the pharmaceutical combination defined in the present application.

### Pharmaceutical Combination

The pharmaceutical combination of the present application comprises: a) a prophylactically and/or therapeutically effective amount of ascorbic acid or a derivative thereof; and
b) a prophylactically and/or therapeutically effective amount of a selective PDE4 inhibitor. The pharmaceutical combination of the present application may further comprise a prophylactically and/or therapeutically effective amount of

platinum compound.

In the present application, the selective PDE4 inhibitor may comprise substances capable of at least partially destroying or blocking the activity of one or more members of the PDE4 subfamily.

For example, the members of the PDE4 subfamily may comprise PDE4A, PDE4B, PDE4C, and/or PDE4D.

For example, the selective PDE4 inhibitor may comprise a substance that at least partially destroys or blocks the activity of PDE4A, PDE4B, PDE4C, or PDE4D.

For example, the selective PDE4 inhibitor may comprise a substance that at least partially destroys or blocks the activities of PDE4C and PDE4D. For example, the selective PDE4 inhibitor may comprise a substance that at least partially destroys or blocks the activities of PDE4B and PDE4C. For example, the selective PDE4 inhibitor may comprise a substance that at least partially destroys or blocks the activities of PDE4B and PDE4D. For example, the selective PDE4 inhibitor may comprise a substance that at least partially destroys or blocks the activities of PDE4A and PDE4B. For example, the selective PDE4 inhibitor may comprise a substance that at least partially destroys or blocks the activities of PDE4A and PDE4C. For example, the selective PDE4 inhibitor may comprise a substance that at least partially destroys or blocks the activities of PDE4A and PDE4D.

For example, the selective PDE4 inhibitor may comprise a substance that at least partially destroys or blocks the activities of PDE4A, PDE4B and PDE4C. For example, the selective PDE4 inhibitor may comprise a substance that at least partially destroys or blocks the activities of PDE4A, PDE4C and PDE4D. For example, the selective PDE4 inhibitor may comprise a substance that at least partially destroys or blocks the activities of PDE4A, PDE4B and PDE4D. For example, the selective PDE4 inhibitor may comprise a substance that at least partially destroys or blocks the activities of PDE4B, PDE4C and PDE4D.

For example, the selective PDE4 inhibitor may comprise a substance that at least partially destroys or blocks the activities of PDE4A, PDE4B, PDE4C and PDE4D.

For example, the at least partially destroying or blocking may comprise destroying or blocking by at least 30%, 40%, 50%, 55%, 60%, 65%, 70%, 75%, 80%, 85%, 90%, or 95%.

For example, the selective PDE4 inhibitor does not substantially destroy or block the activities of other members of the PDEs family (except PDE4). For example, the substantially not destroying or blocking may comprise destroying or blocking by up to 30%, 25%, 20%, 18%, 15%, 13%, 10%, 8%, 5%, 3%, or 1%.

For example, other members of the PDEs family may include members of the PDE1 subfamily, the PDE2 subfamily, the PDE3 subfamily, the PDE5 subfamily, the PDE6 subfamily, the PDE7 subfamily, the PDE8 subfamily, the PDE9 subfamily, the PDE10 subfamily, the PDE11 subfamily, and the PDE12 subfamily.

For example, the activity may include the activity of hydrolyzing cAMP and/or cGMP. For example, the activity of hydrolyzing cAMP and/or cGMP can be determined by any method known to those skilled in the art.

For example, the selective PDE4 inhibitor may comprise apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

For example, the selective PDE4 inhibitor comprises a compound represented by Formula I or a pharmaceutically acceptable salt thereof,

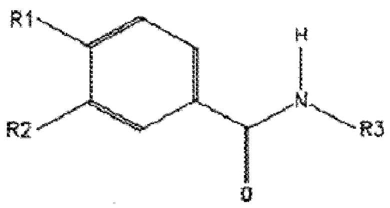

(I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and

R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,

R32 is hydrogen, hydroxy, halogen, amino, trifluoromethyl, $C_1$-$C_4$ alkyl, or $C_1$-$C_4$ alkoxy,

R33 is hydrogen, halogen, $C_1$-$C_4$ alkyl, or $C_1$-$C_4$ alkoxy,

R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl,

R36 is hydrogen or halogen, and

R37 is hydrogen or halogen.

For example, the selective PDE4 inhibitor comprises roflumilast or a derivative thereof.

For example, the derivative of the roflumilast comprises benzanilide and/or bezafibrate.

For example, an effective amount ratio of the selective PDE4 inhibitor to the ascorbic acid or the derivative thereof is about 1:10 to about 1:5,000.

For example, the ratio of the effective amount of the selective PDE4 inhibitor to the effective amount of the ascorbic acid or the derivative thereof is about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1,000, about 1:10 to about 1:1500, about 1:10 to about 1:2,000, about 1:10 to about 1:3,000, about 1:10 to about 1:4,000, about 1:10 to about 1:5,000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1,000, about 1:25 to about 1:5,000, about 1:50 to about 1:100, about 1:50 to about 1:150, about 1:50 to about 1:200, about 1:50 to about 1:300, about 1:50 to about 1:500, about 1:50 to about 1:1,000, about 1:50 to about 1:2,000, about 1:50 to about 1:4,000, or about 1:50 to about 1:5,000, about 1:4500 to about 1:5,000, about 1:100 to about 1:2,000, about 1:1,000 to about 1:4,000, about 1:1,000 to about 1:3,000, about 1:500 to about 1:5,000, or about 1:500 to about 1:2,000.

For example, an effective amount ratio of the selective PDE4 inhibitor to the ascorbic acid or the derivative thereof is about 1:50 to about 1:500.

For example, the effective amount ratio may comprise an effective amount mole ratio and/or an effective amount mass ratio.

For example, a mass ratio of the selective PDE4 inhibitor to the ascorbic acid or the derivative thereof is about 5,000:1 to about 1:5,000.

For example, the mass ratio of the selective PDE4 inhibitor to the ascorbic acid or the derivative thereof is about 20:1 to 1:1, about 20:1 to about 5:1, about 20:1 to about 10:1, about 25:1 to about 10:1, about 50:1 to about 10:1, about 100:1 to about 10:1, about 150:1 to about 10:1, about 200:1 to about 10:1, about 250:1 to about 10:1, about 300:1 to about 10:1, about 400:1 to about 10:1, about 450:1 to about 10:1, about 10:1 to about 500:1, about 10:1 to about 550:1, about 10:1 to about 600:1, about 10:1 to about 650:1, about 700:1 to about 10:1, about 800:1 to about 10:1, about 900:1 to about 10:1, about 1,000:1 to about 10:1, about 1500:1 to about 10:1, about 2,000:1 to about 10:1, about 3,000:1 to about 10:1, about 4,000:1 to about 10:1, about 5,000:1 to about 10:1, about 20:1 to about 5:1 about 1,000:1 to about 50:1, about 2,000:1 to about 50:1, about 3,000:1 to about 50:1, about 4,000:1 to about 50:1, about 5,000:1 to about 1:50, about 20:1 to 1:20, about 20:1 to about 1:20, about 20:1 to about 1:25, about 25:1 to about 50:1, about 50:1 to about 1:100, about 100:1 to about 1:100, about 150:1 to about 1:100, about 200:1 to about 1:200, about 250:1 to about 1:500, about 300:1 to about 1:1,000, about 400:1 to about 1:100, about 450:1 to about 1:500, about 10:1 to about 1:500, about 10:1 to about 1:550, about 10:1 to about 1:600, about 10:1 to about 1:650, about 700:1 to about 1:650, about 800:1 to about 1:800, about 900:1 to about 1:900, about 1,000:1 to about 1:1,000, about 1500:1 to about 1:1,000. about 2,000:1 to about 1:1500, about 3,000:1 to about 1:500, about 4,000:1 to about 1:2500, about 5,000:1 to about 1:3500, about 20:1 to about 1:1500, about 1,000:1 to about 1:2,000, about 2,000:1 to about 1:5,000 about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1,000, about 1:10 to about 1:1500, about 1:10 to about 1:2,000, about 1:10 to about 1:3,000, about 1:10 to about 1:4,000, about 1:10 to about 1:5,000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1,000, about 1:25 to about 1:5,000, about 1:50 to about 1:100, about 1:50 to about 1:150, about 1:50 to about 1:200, about 1:50 to about 1:300, about 1:50 to about 1:500, about 1:50 to about 1:1,000, about 1:50 to about 1:2,000, about 1:50 to about 1:3,000, about 1:50 to about 1:4,000, or about 1:50 to about 1:5,000.

For example, a mass ratio of the selective PDE4 inhibitor to the ascorbic acid or the derivative thereof is about 1:50 to about 1:500.

For example, a mass ratio of the selective PDE4 inhibitor to the ascorbic acid or the derivative thereof is about 1:300 to about 1:500.

For example, an effective amount ratio of the selective PDE4 inhibitor to the ascorbic acid or the derivative thereof to the platinum compound is about 1:10:0.1 to about 1:5,000:10.

For example, the effective amount ratio of the selective PDE4 inhibitor to the ascorbic acid or the derivative thereof to the platinum compound is about 1:1:0.1 to about 1:20:1, about 1:5:0.5 to about 1:20:1, about 1:10:0.1 to about 1:20:0.5, about 1:10:2 to about 1:25:0.5, about 1:10:0.5 to about 1:50:0.5, about 1:10:0.5 to about 1:100:0.5, about 1:10:0.5 to about 1:150:0.5, about 1:10:0.5 to about 1:200:0.5, about 1:10 to about 1:250:0.5, about 1:10:0.5 to about 1:300:0.5, about 1:10 to about 1:400:0.5, about 1:10:1 to about 1:450:1, about 1:10:2 to about 1:500:0.5, about 1:10:2 to about 1:550:2, about 1:10:0.5 to about 1:600:5, about 1:10:0.5 to about 1:650:1, about 1:10:1 to about 1:700:5, about 1:10:1 to about 1:800:2, about 1:10:0.5 to about 1:900:5, about 1:10:0.5 to about 1:1,000:1, about 1:100:1 to about 1:1500:3, about 1:10:0.1 to about 1:2,000:1, about 1:10:0.1 to about 1:3,000:2, about 1:10:1 to about 1:4,000:1, about 1:10:0.5 to about 1:5,000:1, about 1:5:0.5 to about 1:20:1, about 1:5:0.5 to about 1:25:1, about 1:50.5 to about 1:35:1, about 1:5:0.5 to about 1:50:1, about 1:5:0.5 to about 1:100:1, about 1:5:1 to about 1:150:2, about 1:5:0.5 to about 1:200:1, about 1:25:1 to about 1:50:1, about 1:25:1 to about 1:100:1, about 1:25:1 to about 1:150:2, about 1:25:1 to about 1:200:5, about 1:25:2 to about 1:1,000:2, about 1:25:0.5 to about 1:5,000:1, about 1:50:0.5 to about 1:100:1, about 1:50:0.5 to about 1:150:2, about 1:50:0.5 to about 1:200:2, about 1:50:0.5 to about 1:300:5, about 1:50:0.5 to about 1:500:5, about 1:50:0.5 to about 1:1,000:5, about 1:50:0.5 to about 1:2,000:10, about 1:50:1 to about 1:4,000:10 or about 1:50:5 to about 1:5,000:10, about 1:4500:1 to about 1:5,000:5, about 1:100:1 to about 1:2,000:5, about 1:1,000:0.5 to about 1:4,000:5, about 1:1,000:0.5 to about 1:3,000:5, about 1:500:1 to about 1:5,000:1, about 1:500:1 to about 1:2,000:1.

For example, an effective amount ratio of the selective PDE4 inhibitor to the ascorbic acid or the derivative thereof to the platinum compound is about 1:50:1 to about 1:500:1.

For example, an effective amount ratio of the selective PDE4 inhibitor to the ascorbic acid or the derivative thereof to the platinum compound is about 1:300:1 to about 1:500:1.

For example, the selective PDE4 inhibitor, the ascorbic acid or the derivative thereof, and the platinum compound may each be from a different source, for example, manufactured or sold by different merchants. For example, the ascorbic acid or the derivative thereof prepared, manufactured or sold is not necessarily single-component or pure, as long as it contains a compound, or a solvate, hydrate, or salt thereof, which is capable of releasing the ascorbic acid (vitamin C) in vivo or in vitro, and/or contains ascorbic acid analogs (for example, in which one or more hydroxyl groups of the ascorbic acid are substituted by another structural moiety, and the ascorbic acid analogs substantially retains the stable activity of the ascorbic acid in vitro or in vivo), which are all within the scope of the present application. In a similar way, the selective PDE4 inhibitors or platinum compounds prepared, manufactured or sold are not necessarily single-component or pure, as long as they contains any one or more of the selective PDE4 inhibitors or platinum compounds of the present application, or solvates, hydrates, or salts thereof, which can be released in vivo or in vitro, and/or contains the analogs (for example, which comprise group modification or substitution, and which substantially maintain the stable activity of any selective PDE4 inhibitor or platinum compound in vitro or in vivo) of any one or more of the selective PDE4 inhibitors or platinum compounds, which are all within the scope of the present application.

For example, the selective PDE4 inhibitor and the ascorbic acid or the derivative thereof are each present in a different container, respectively.

For example, the selective PDE4 inhibitor and the platinum compound are present in the same container, and the selective PDE4 inhibitor and the ascorbic acid or the derivative thereof are each present in a different container, respectively.

For example, the ascorbic acid or the derivative thereof and the platinum compound are present in the same container, and the ascorbic acid or the derivative thereof and the selective PDE4 inhibitor are each present in a different container, respectively.

For example, the selective PDE4 inhibitor, the ascorbic acid or the derivative thereof, and the platinum compound are each present in a different container, respectively.

For example, the amount of the selective PDE4 inhibitor, the amount of the ascorbic acid or the derivative thereof, and the amount of the platinum compound may be set up according to their effective amount ratio, or may not be set up according to their effective amount ratio.

For example, the selective PDE4 inhibitor may be present in one or more containers, for example, 2 or more, 3 or more, 4 or more, 5 or more, 6 or more, 7 or more, 8 or more, 9 or more, 10 or more, 11 or more, or 12 or more contains.

For example, the ascorbic acid or the derivative thereof may be present in 1 or more containers, for example, 2 or more, 3 or more, 4 or more, 5 or more, 6 or more, 7 or more, 8 or more, 9 or more, 10 or more, 11 or more, or 12 or more containers.

For example, the platinum compound may be present in one or more containers, for example, 2 or more, 3 or more, 4 or more, 5 or more, 6 or more, 7 or more, 8 or more, 9 or more, 10 or more, 11 or more, or 12 or more containers.

For example, the container may comprise a medicine case, a medicine box, a medicine bottle, a medicine bag, a blister, a tube, a syringe, etc. For example, the medicine bottle may comprise a sealed glass ampoule, a test tube, a vial, a flask, a bottle, etc. For example, the container may be made of glass, polycarbonate, polystyrene and other organic polymers, or ceramics, metals, composite films, cellophane, packaging materials internally lined with foils such as aluminum or alloy, and any other appropriate materials that are generally used to hold reagents.

For example, the selective PDE4 inhibitor, the platinum compound, and the ascorbic acid or the derivative thereof may each be present in a different container, and when needed, may be formulated into a formulation with a suitable carrier simultaneously or separately.

For example, the selective PDE4 inhibitor, the platinum compound, and the ascorbic acid or the derivative thereof are mixed to formulate an appropriate formulation.

For example, the selective PDE4 inhibitor, the platinum compound, and the ascorbic acid or the derivative thereof are each formulated into a different appropriate formulation.

For example, the selective PDE4 inhibitor, the platinum compound, and the ascorbic acid or the derivative thereof may each be present in the form of a formulation in a different container.

For example, the pharmaceutical combination comprises a first formulation and a second formulation, and the first formulation comprises the ascorbic acid or the derivative thereof and a first pharmaceutically acceptable carrier, and the second formulation comprises the selective PDE4 inhibitor and a second pharmaceutically acceptable carrier.

For example, the pharmaceutical combination comprises a first formulation, a second formulation, and a third formulation, the first formulation comprises the ascorbic acid or the derivative thereof and a first pharmaceutically acceptable carrier, the second formulation comprises the selective PDE4 inhibitor and a second pharmaceutically acceptable carrier, and the third formulation comprises the platinum compound and a third pharmaceutically acceptable carrier.

For example, the first formulation and the second formulation may have the same dosage form or different dosage forms.

For example, the second formulation and the third formulation may have the same dosage form or different dosage forms.

For example, the first formulation and the third formulation may have the same dosage form or different dosage forms.

For example, the first formulation, the second formulation, and the third formulation may have the same dosage form.

For example, the first formulation, the second formulation, and the third formulation may have dosage forms different from one another.

For example, the first formulation, the second formulation, and the third formulation may all be any one of the dosages forms such as pills, powders, tablets, granules, gels, hard capsules, soft capsules, syrups, admixtures, lotions, injections, aerosols, ointments, films, suppositories, and dripping pills, and may also be any combination thereof. For example, the first formulation, the second formulation, and the third formulation are all injections. For example, the first formulation and the second formulation are tablets, and the third formulation is an injection. For example, the first formulation and the third formulation are injections, and the second formulation is a tablet. For example, the second formulation and the third formulation are injections, and the first formulation is a tablet. For example, the first formulation is a tablet, the second formulation is a granule, and the third formulation is an injection.

For example, the first formulation, the second formulation, and the third formulation may exist in the form of a formulation adapted to the same method of administration. For example, the first formulation, the second formulation, and the third formulation may all be tablets, granules, hard capsules, soft capsules, or syrups adapted to oral administration. For example, the first formulation, the second formulation, and the third formulation may all be injections adapted to injection.

For example, the first formulation, the second formulation, and the third formulation may exist in the form of formulations adapted to different methods of administration. For example, the first formulation and the second formulation may be tablets, granules, hard capsules, soft capsules, or syrups adapted to oral administration, and the third formulations may be an injection adapted to injection. For example, the first formulation and the third formulation may be injections adapted to injection, and the second formulation may be an oral tablet, a granule, a hard capsule, a soft capsule, or a syrup. For example, the second formulation and the third formulation may be injections adapted to injection, and the first formulation may be an oral tablet, a granule, a hard capsule, a soft capsule, or a syrup. For example, the first formulation may be an injection adapted to injection, and the second formulation may be an oral tablet, a granule, a hard capsule, a soft capsule, or a syrup, and the third formulation may be a sustained-release formulation which is encapsulated in a microcapsule and which is adapted to encapsulation and sustained-release.

For example, the pharmaceutical combination comprises a pharmaceutical composition, which comprises the selective PDE4 inhibitor and the ascorbic acid or the derivative thereof.

For example, the pharmaceutical composition further comprises a platinum compound.

For example, the selective PDE4 inhibitor in the pharmaceutical composition has a content of about 0.01% to about 20% (w/w).

For example, the selective PDE4 inhibitor in the pharmaceutical composition has a content of about 0.01% (w/w) to about 0.5% (w/w), about 0.05% (w/w) to about 5% (w/w), about 0.5% (w/w) to about 5% (w/w), about 10% (w/w) to about 20% (w/w), about 1% (w/w) to about 10% (w/w), about 5% (w/w) to about 20% (w/w), about 0.2% (w/w) to about 4% (w/w), about 0.1% (w/w) to about 5% (w/w), about 3% (w/w) to about 20% (w/w), about 5% to about 20% (w/w), or

about 1% (w/w) to about 15% (w/w).

For example, the selective PDE4 inhibitor in the pharmaceutical composition has a content of about 1% to about 5% (w/w).

For example, the ascorbic acid or the derivative thereof in the pharmaceutical composition has a content of about 30% to about 99% (w/w).

For example, the ascorbic acid or the derivative thereof has a content of about 30% (w/w) to about 60% (w/w), about 40% (w/w) to about 60% (w/w), about 30% (w/w) to about 50% (w/w), about 50% (w/w) to about 60% (w/w), about 50% (w/w) to about 70% (w/w), about 60% (w/w) to about 80% (w/w), about 50% (w/w) to about 90% (w/w), about 60% (w/w) to about 90% (w/w), or about 55% (w/w) to about 75% (w/w), about 70% (w/w) to about 90% (w/w), or about 80% to about 95% (w/w).

For example, the ascorbic acid or the derivative thereof in the pharmaceutical composition has a content of about 90% to about 98% (w/w).

For example, the platinum compound in the pharmaceutical composition has a content of about 0.01% (w/w) to about 0.5% (w/w), about 0.05% (w/w) to about 5% (w/w), about 0.5% (w/w) to about 5% (w/w), about 10% (w/w) to about 20% (w/w), about 1% (w/w) to about 10% (w/w), about 5% (w/w) to about 20% (w/w), about 0.2% (w/w) to about 4% (w/w), about 0.1% (w/w) to about 5% (w/w), about 3% (w/w) to about 20% (w/w), about 5% to about 20% (w/w), or about 1% (w/w) to about 15% (w/w).

For example, the platinum compound in the pharmaceutical composition has a content of about 1% to about 5% (w/w).

**Kit**

The kit of the present application may comprise the pharmaceutical combination of the present application. The pharmaceutical combination may be provided in the form of a kit, and different constituents in the pharmaceutical combination may be packaged in different containers, mixed before administration, or administered separately without mixing.

For example, separate packaging may be for the purpose of long-term storage without losing the functions of active constituents.

For example, the formulation contained in the kit may be present in any kind of container in which the ingredients of the formulations effectively maintain their activity for a long time, are not adsorbed by the material of the container, and are not easily deteriorated. For instance, in the case of a sealed glass ampoule, for example, the ampoule may be made of organic polymers (such as glass, polycarbonate, and polystyrene), ceramics, metals, or any other suitable materials that can be generally used to hold reagents. Examples of other suitable containers include simple bottles made of substances similar to those of ampoules, and packaging materials internally lined with foils such as aluminum or alloy. Other containers include test tubes, vials, flasks, bottles, syringes, or the like. The container is provided with a sterile access port of a bottle or the like, and the bottle is provided with a stopper that can be penetrated through by a hypodermic syringe needle.

For example, the kit may also comprise a buffer that is packaged in the presence of a neutral and non-reactive gas such as nitrogen.

For example, the kit may also comprise an auxiliary administration means, for example, a measuring cup and a measuring spoon adapted to oral administration, and for example, a syringe, an infusion tube, an infusion needle and others adapted to injection.

For example, the kit is also included with instructions for use. The instructions for use of the kit consisting of the pharmaceutical combination may be printed on paper or other materials, and/or may be supplied as a Floppy (registered trademark) disk, CD-ROM, DVD-ROM, a Zip disk, a video tape, an audio tape, or other media that is readable electrically or electromagnetically. The detailed instructions for use may be actually attached inside the kit, or posted on a website designated by the manufacturer or distributor of the kit or notified by means of e-mail or the like.

For example, the pharmaceutical combination provided in the present application may be recorded in the instructions for use.

For example, the use or method provided in the present application may be recorded in the instructions for use.

**Uses and Methods**

The present application provides use of a combination of ascorbic acid or a derivative thereof and a selective PDE4 inhibitor in the preparation of a medicament for preventing and/or treating a tumor. The present application further provides use of a combination of ascorbic acid or a derivative thereof, a selective PDE4 inhibitor, and the platinum compound in the preparation of a medicament for preventing and/or treating a tumor.

The present application further provides a method for preventing and/or treating a tumor, comprising administering:

a) an effective amount of ascorbic acid or a derivative thereof; and
b) an effective amount of a selective PDE4 inhibitor, to a subject in need thereof.

For example, the method further comprises administering an effective amount of platinum compound to a subject in need thereof.
For example, the tumor comprises a solid tumor and/or a non-solid tumor.
For example, the solid tumor may be selected from the group consisting of: a liver cancer, a gastric cancer, a lung cancer, a breast cancer, a colon cancer, a rectal cancer, a renal cell cancer, a liver cancer, a non-small cell lung cancer, a small intestine cancer, an esophageal cancer, melanoma, a bone cancer, a pancreatic cancer, a skin cancer, a head or neck cancer, a skin or intraocular malignant melanoma, a uterine cancer, an ovarian cancer, a rectal cancer, a testicular cancer, a fallopian tube cancer, an endometrial cancer, a cervical cancer, a vaginal cancer, a vulvar cancer, a Hodgkin's disease, a non-Hodgkin's lymphoma, a endocrine system cancer, a thyroid cancer, a parathyroid cancer, an adrenal cancer, a soft tissue sarcoma, a urethral cancer, a penile cancer, a solid tumor in children, a bladder cancer, a kidney or ureter cancer, a renal pelvis cancer, a central nervous system (CNS) tumor, a primary CNS lymphoma, tumor angiogenesis, a spinal tumor, a brain stem glioma, a pituitary adenoma, a Kaposi sarcoma, an epidermoid carcinoma, a squamous cell carcinoma, a T-cell lymphoma, and metastatic foci of these cancers.
For example, the non-solid tumor may be selected from the group consisting of: chronic lymphocytic leukemia (CLL), acute leukemia, acute lymphoid leukemia (ALL), B-cell acute lymphoid leukemia (B-ALL), T-cell acute lymphoid leukemia (T-ALL), chronic myelogenous leukemia (CML), acute myelogenous leukemia (AML), B-cell prolymphocytic leukemia, a blastic plasmacytoid dendritic cell tumor, Burkitt lymphoma, diffuse large B-Cell lymphoma, follicular lymphoma, hairy cell leukemia, small cell or large cell follicular lymphoma, a malignant lymphoproliferative disease, MALT lymphoma, mantle cell lymphoma, marginal zone lymphoma, multiple myeloma, myelodysplasia and myelodysplastic syndrome, non-Hodgkin's lymphoma, Hodgkin's lymphoma, plasmablastic lymphoma, plasmacytoid dendritic cell tumor, and Waldenstrom macroglobulinemia.
For example, the tumor comprises a colon cancer and/or melanoma.
For example, the tumor comprises a tumor or tumor cell having no response to the platinum compound.
For example, the selective PDE4 inhibitor and the ascorbic acid or the derivative thereof are formulated to be simultaneously administered to the subject.
For example, the selective PDE4 inhibitor and the ascorbic acid or the derivative thereof are simultaneously administered to the subject.
For example, the simultaneous administration to the subject may comprise that the time interval between the time for administering the selective PDE4 inhibitor and the time for administering the ascorbic acid or the derivative thereof to a subject is not greater than 1 hour. For example, the time interval is 60 minutes, 55 minutes, 50 minutes, 45 minutes, 40 minutes, 35 minutes, 30 minutes, 25 minutes, 20 minutes, 15 minutes, 10 minutes, 8 minutes, 5 minutes, 3 minutes, 2 minutes, 1 minute, or the administration is conducted in a mixed form.
For example, the selective PDE4 inhibitor and the ascorbic acid or the derivative thereof are formulated to be separately administered to a subject.
For example, the selective PDE4 inhibitor and the ascorbic acid or the derivative thereof are sequentially administered to the subject.
For example, the separate administration to the subject may comprise that the time interval between the time for administering the selective PDE4 inhibitor and the time for administering the ascorbic acid or the derivative thereof to a subject is greater than 1 hour. For example, the time interval is 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 9 hours, 10 hours, 11 hours, 12 hours, 15 hours, 18 hours, 21 hours, 24 hours, 2 days, 3 days, 4 days, 5 days, 6 days, 7 days, or longer.
For example, the sequential administration to the subject may comprise that the time interval between the time for administering the selective PDE4 inhibitor and the time for administering the ascorbic acid or the derivative thereof to a subject is greater than 1 hour. For example, the time interval is 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 9 hours, 10 hours, 11 hours, 12 hours, 15 hours, 18 hours, 21 hours, 24 hours, 2 days, 3 days, 4 days, 5 days, 6 days, 7 days, or longer.
For example, the selective PDE4 inhibitor, the ascorbic acid or the derivative thereof, and the platinum compound are formulated to be simultaneously administered to the subject.
For example, the selective PDE4 inhibitor, the ascorbic acid or the derivative thereof, and the platinum compound are simultaneously administered to the subject.
For example, the simultaneous administration to the subject may comprise administering the selective PDE4 inhibitor, the ascorbic acid or the derivative thereof, and the platinum compound together in the form of a mixture.
For example, the selective PDE4 inhibitor, the ascorbic acid or the derivative thereof, and the platinum compound

are formulated to be each administered to the subject in any order, and the order of administration may comprise the selective PDE4 inhibitor, the ascorbic acid or the derivative thereof, and the platinum compound; or the selective PDE4 inhibitor, the platinum compound, and the ascorbic acid or the derivative thereof; or the ascorbic acid or the derivative thereof, the selective PDE4 inhibitor, and the platinum compound; or, the ascorbic acid or the derivative thereof, the platinum compound, or the selective PDE4 inhibitor; or, the platinum compound, the ascorbic acid or the derivative thereof, and the selective PDE4 inhibitor; or the platinum compound, the selective PDE4 inhibitor, and the ascorbic acid or the derivative thereof. The simultaneous administration to the subject may comprise that, among the selective PDE4 inhibitor, the ascorbic acid or the derivative thereof, and the platinum compound, the time interval between the time for administering the first one to the subject and the time for administering the last one to the subject is not greater than 1 hour. For example, the time interval is 60 minutes, 55 minutes, 50 minutes, 45 minutes, 40 minutes, 35 minutes, 30 minutes, 25 minutes, 20 minutes, 15 minutes, 10 minutes, 8 minutes, 5 minutes, 3 minutes, 2 minutes, or 1 minute.

For example, the selective PDE4 inhibitor, the ascorbic acid or the derivative thereof, and the platinum compound are formulated to be separately administered to a subject.

For example, the selective PDE4 inhibitor, the ascorbic acid or the derivative thereof, and the platinum compound are sequentially administered to the subject.

For example, the selective PDE4 inhibitor, the ascorbic acid or the derivative thereof, and the platinum compound are formulated to be each administered to the subject in any order, and the order of administration may comprise the selective PDE4 inhibitor, the ascorbic acid or the derivative thereof, and the platinum compound; or the selective PDE4 inhibitor, the platinum compound, and the ascorbic acid or the derivative thereof; or the ascorbic acid or the derivative thereof, the selective PDE4 inhibitor, and the platinum compound; or, the ascorbic acid or the derivative thereof, the platinum compound, or the selective PDE4 inhibitor; or, the platinum compound, the ascorbic acid or the derivative thereof, and the selective PDE4 inhibitor; or the platinum compound, the selective PDE4 inhibitor, and the ascorbic acid or the derivative thereof. The separate administration to the subject may comprise that, among the selective PDE4 inhibitor, the ascorbic acid or the derivative thereof, and the platinum compound, the time interval between the time for administering the first one to the subject and the time for administering the last one to the subject is greater than 1 hour. For example, the time interval is 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 9 hours, 10 hours, 11 hours, 12 hours, 15 hours, 18 hours, 21 hours, 24 hours, 2 days, 3 days, 4 days, 5 days, 6 days, 7 days, or longer. The sequential administration to the subject may comprise that, among the selective PDE4 inhibitor, the ascorbic acid or the derivative thereof, and the platinum compound, the time interval between the time for administering the first one to the subject and the time for administering the last one to the subject is greater than 1 hour. For example, the time interval is 1.5 hours, 2 hours, 2.5 hours, 3 hours, 35 hours, 4 hours, 4.5 hours, 5 hours, 5.5 hours, 6 hours, 6.5 hours, 7 hours, 7.5 hours, 8 hours, 9 hours, 10 hours, 11 hours, 12 hours, 15 hours, 18 hours, 21 hours, 24 hours, 2 days, 3 days, 4 days, 5 days, 6 days, 7 days, or longer.

For example, the administration of the selective PDE4 inhibitor may comprise one administration.

For example, the administration of the selective PDE4 inhibitor may comprise 2 or more consecutive administrations, for example, 3 or more, 4 or more, 5 or more, 6 or more, 7 or more, 8 or more, 9 or more, or 10 or more consecutive administrations, and then the ascorbic acid or the derivative thereof or the platinum compound is administered.

For example, the administration of the ascorbic acid or the derivative thereof may comprise one administration.

For example, the administration of the ascorbic acid or the derivative thereof may comprise 2 or more consecutive administrations, for example, 3 or more, 4 or more, 5 or more, 6 or more, 7 or more, 8 or more, 9 or more, or 10 or more consecutive administrations, and then the selective PDE4 inhibitor or the platinum compound is administered.

For example, the administration of the platinum compound may comprise one administration.

For example, the administration of the platinum compound may comprise 2 or more consecutive administrations, for example, 3 or more, 4 or more, 5 or more, 6 or more, 7 or more, 8 or more, 9 or more, or 10 or more consecutive administrations, and then the selective PDE4 inhibitor or the platinum compound is administered.

For example, the medicament is formulated such that the selective PDE4 inhibitor is administered at a dose of about 0.05 mg/kg to about 40 mg/kg.

For example, the selective PDE4 inhibitor is administered at a dose of about 0.05 mg/kg to about 40 mg/kg.

For example, the selective PDE4 inhibitor is administered at a dose of about 1 mg/kg to about 30 mg/kg, about 1 mg/kg to about 20 mg/kg, about 1 mg/kg to about 40 mg/kg, about 5 mg/kg to about 20 mg/kg, about 10 mg/kg to about 40 mg/kg, about 5 mg/kg to about 30 mg/kg, about 0.1 mg/kg to about 30 mg/kg, about 0.5 mg/kg to about 20 mg/kg, about 0.5 mg/kg to about 30 mg/kg, about 10 mg/kg to about 30 mg/kg, or about 20 mg/kg to about 40 mg/kg.

For example, the selective PDE4 inhibitor is administered at a dose of about 1 mg/kg to about 30 mg/kg, about 1 mg/kg to about 20 mg/kg, about 1 mg/kg to about 40 mg/kg, about 5 mg/kg to about 20 mg/kg, about 10 mg/kg to about 40 mg/kg, about 5 mg/kg to about 30 mg/kg, about 0.1 mg/kg to about 30 mg/kg, about 0.5 mg/kg to about 20 mg/kg, about 0.5 mg/kg to about 30 mg/kg, about 10 mg/kg to about 30 mg/kg, or about 20 mg/kg to about 40 mg/kg.

For example, the medicament is formulated such that the selective PDE4 inhibitor is administered at a dose of

about 1 mg/kg to about 10 mg/kg.

For example, the selective PDE4 inhibitor is administered at a dose of about 1 mg/kg to about 10 mg/kg.

For example, the medicament is formulated such that the ascorbic acid or the derivative thereof is administered at a dose of about 0.05 g/kg to about 2.5 g/kg.

For example, the ascorbic acid or the derivative thereof is administered at a dose of about 0.05 g/kg to about 2.5 g/kg.

For example, the ascorbic acid or the derivative thereof is administered at a dose of about 0.05 g/kg to about 0.1 g/kg, about 0.1 g/kg to about 0.2 g/kg, about 0.15 g/kg to about 0.25 g/kg, about 0.05 g/kg to about 0.15 g/kg, about 0.05 g/kg to about 2.5 g/kg, about 0.25 g/kg to about 1 g/kg, about 0.35 g/kg to about 0.5 g/kg, about 0.5 g/kg to about 1 g/kg, about 0.5 g/kg to about 2.5 g/kg, about 0.5 g/kg to about 2 g/kg, about 1 g/kg to about 2 g/kg, about 1 g/kg to about 2.5 g/kg, about 1.5 g/kg to about 2.5 g/kg, about 0.1 g/kg to about 2.5 g/kg, about 0.5 g/kg to about 2 g/kg, or about 0.05 g/kg to about 0.2 g/kg.

For example, the ascorbic acid or the derivative thereof is administered at a dose of about 0.05 g/kg to about 0.1 g/kg. about 0.1 g/kg to about 0.2 g/kg. about 0.15 g/kg to about 0.25 g/kg, about 0.05 g/kg to about 0.15 g/kg, about 0.05 g/kg to about 2.5 g/kg, about 0.25 g/kg to about 1 g/kg, about 0.35 g/kg to about 0.5 g/kg, about 0.5 g/kg to about 1 g/kg, about 0.5 g/kg to about 2.5 g/kg, about 0.5 g/kg to about 2 g/kg, about 1 g/kg to about 2 g/kg, about 1 g/kg to about 2.5 g/kg, about 1.5 g/kg to about 2.5 g/kg, about 0.1 g/kg to about 2.5 g/kg, about 0.5 g/kg to about 2 g/kg, or about 0.05 g/kg to about 0.2 g/kg.

For example, the medicament is formulated such that the ascorbic acid or the derivative thereof is administered at a dose of about 0.5 g/kg to about 2 g/kg.

For example, the ascorbic acid or the derivative thereof is administered at a dose of about 0.5 g/kg to about 2 g/kg.

For example, the medicament is formulated such that the platinum compound is administered at a dose of about 0.05 mg/kg to about 40 mg/kg.

For example, the platinum compound is administered at a dose of about 0.05 mg/kg to about 40 mg/kg.

For example, the platinum compound is administered at a dose of about 1 mg/kg to about 30 mg/kg, about 1 mg/kg to about 20 mg/kg, about 1 mg/kg to about 40 mg/kg, about 5 mg/kg to about 20 mg/kg, about 10 mg/kg to about 40 mg/kg, about 5 mg/kg to about 30 mg/kg, about 0.1 mg/kg to about 30 mg/kg, about 0.5 mg/kg to about 20 mg/kg, about 0.5 mg/kg to about 30 mg/kg, about 10 mg/kg to about 30 mg/kg, or about 20 mg/kg to about 40 mg/kg.

For example, the platinum compound is administered at a dose of about 1 mg/kg to about 30 mg/kg, about 1 mg/kg to about 20 mg/kg, about 1 mg/kg to about 40 mg/kg, about 5 mg/kg to about 20 mg/kg, about 10 mg/kg to about 40 mg/kg, about 5 mg/kg to about 30 mg/kg, about 0.1 mg/kg to about 30 mg/kg, about 0.5 mg/kg to about 20 mg/kg, about 0.5 mg/kg to about 30 mg/kg, about 10 mg/kg to about 30 mg/kg, or about 20 mg/kg to about 40 mg/kg.

For example, the medicament is formulated such that the platinum compound is administered at a dose of about 1 mg/kg to about 10 mg/kg.

For example, the platinum compound is administered at a dose of about 1 mg/kg to about 10 mg/kg.

For example, the medicament is formulated such that the selective PDE4 inhibitor and the ascorbic acid or the derivative thereof are administered at a mass ratio of 1:5 to about 1:5,000.

For example, the selective PDE4 inhibitor and the ascorbic acid or the derivative thereof are administered at a mass ratio of about 1:5 to about 1:5,000.

For example, the medicament is formulated such that the selective PDE4 inhibitor and the ascorbic acid or the derivative thereof are administered at a mass ratio of about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1,000, about 1:10 to about 1:1500, about 1:10 to about 1:2,000, about 1:10 to about 1:3,000, about 1:10 to about 1:4,000, about 1:10 to about 1:5,000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1,000, about 1:25 to about 1:5,000, about 1:50 to about 1:100, about 1:50 to about 1:150, about 1:50 to about 1:200, about 1:50 to about 1:300, about 1:50 to about 1:500, about 1:50 to about 1:1,000, about 1:50 to about 1:2,000, about 1:50 to about 1:4,000, or about 1:50 to about 1:5,000, about 1:4500 to about 1:5,000, about 1:100 to about 1:2,000, about 1:1,000 to about 1:4,000, about 1:1,000 to about 1:3,000, about 1:500 to about 1:5,000, or about 1:500 to about 1:2,000.

For example, the selective PDE4 inhibitor and the ascorbic acid or the derivative thereof are administered at a mass ratio of about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about

1:10 to about 1:800, about 1:10 to about 1:900, about 1:10 to about 1:1,000, about 1:10 to about 1:1500, about 1:10 to about 1:2,000, about 1:10 to about 1:3,000, about 1:10 to about 1:4,000, about 1:10 to about 1:5,000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1,000, about 1:25 to about 1:5,000, about 1:50 to about 1:100, about 1:50 to about 1:150, about 1:50 to about 1:200, about 1:50 to about 1:300, about 1:50 to about 1:500, about 1:50 to about 1:1,000, about 1:50 to about 1:2,000, about 1:50 to about 1:4,000, or about 1:50 to about 1:5,000, about 1:4500 to about 1:5,000, about 1:100 to about 1:2,000, about 1:1,000 to about 1:4,000, about 1:1,000 to about 1:3,000, about 1:500 to about 1:5,000, or about 1:500 to about 1:2,000.

For example, the medicament is formulated such that the selective PDE4 inhibitor and the ascorbic acid or the derivative thereof are administered at a mass ratio of 1:50 to about 1:500.

For example, the selective PDE4 inhibitor and the ascorbic acid or the derivative thereof are administered at a mass ratio of about 1:50 to about 1:500.

For example, the medicament is formulated such that the selective PDE4 inhibitor and the ascorbic acid or the derivative thereof are administered at an effective amount ratio of about 1:5 to about 1:5,000.

For example, the selective PDE4 inhibitor and the ascorbic acid or the derivative thereof are administered at an effective amount ratio of about 1:5 to about 1:5,000.

For example, the medicament is formulated such that the selective PDE4 inhibitor and the ascorbic acid or the derivative thereof are administered at an effective amount ratio of about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1,000, about 1:10 to about 1:1500, about 1:10 to about 1:2,000, about 1:10 to about 1:3,000, about 1:10 to about 1:4,000, about 1:10 to about 1:5,000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1,000, about 1:25 to about 1:5,000, about 1:50 to about 1:100, about 1:50 to about 1:150, about 1:50 to about 1:200, about 1:50 to about 1:300, about 1:50 to about 1:500, about 1:50 to about 1:1,000, about 1:50 to about 1:2,000, about 1:50 to about 1:4,000, or about 1:50 to about 1:5,000, about 1:4500 to about 1:5,000, about 1:100 to about 1:2,000, about 1:1,000 to about 1:4,000, about 1:1,000 to about 1:3,000, about 1:500 to about 1:5,000, or about 1:500 to about 1:2,000.

For example, the selective PDE4 inhibitor and the ascorbic acid or the derivative thereof are administered at an effective amount ratio of about 1:1 to about 1:20, about 1:5 to about 1:20, about 1:10 to about 1:20, about 1:10 to about 1:25, about 1:10 to about 1:50, about 1:10 to about 1:100, about 1:10 to about 1:150, about 1:10 to about 1:200, about 1:10 to about 1:250, about 1:10 to about 1:300, about 1:10 to about 1:400, about 1:10 to about 1:450, about 1:10 to about 1:500, about 1:10 to about 1:550, about 1:10 to about 1:600, about 1:10 to about 1:650, about 1:10 to about 1:700, about 1:10 to about 1:800, about 1:10 to about 1:900, about 1:10 to about 1:1,000, about 1:10 to about 1:1500, about 1:10 to about 1:2,000, about 1:10 to about 1:3,000, about 1:10 to about 1:4,000, about 1:10 to about 1:5,000, about 1:5 to about 1:20, about 1:5 to about 1:25, about 1:5 to about 1:35, about 1:5 to about 1:50, about 1:5 to about 1:100, about 1:5 to about 1:150, about 1:5 to about 1:200, about 1:25 to about 1:50, about 1:25 to about 1:100, about 1:25 to about 1:150, about 1:25 to about 1:200, about 1:25 to about 1:1,000, about 1:25 to about 1:5,000, about 1:50 to about 1:100, about 1:50 to about 1:150, about 1:50 to about 1:200, about 1:50 to about 1:300, about 1:50 to about 1:500, about 1:50 to about 1:1,000, about 1:50 to about 1:2,000, about 1:50 to about 1:4,000, or about 1:50 to about 1:5,000, about 1:4500 to about 1:5,000, about 1:100 to about 1:2,000, about 1:1,000 to about 1:4,000, about 1:1,000 to about 1:3,000, about 1:500 to about 1:5,000, about 1:500 to about 1:2,000.

For example, the medicament is formulated such that the selective PDE4 inhibitor and the ascorbic acid or the derivative thereof are administered at an effective amount ratio of about 1:50 to about 1:500.

For example, the selective PDE4 inhibitor and the ascorbic acid or the derivative thereof are administered at an effective amount ratio of about 1:5 to about 1:5,000.

For example, the medicament is formulated such that the selective PDE4 inhibitor, the ascorbic acid or the derivative thereof, and the platinum compound are administered at a mass ratio (or an effective amount ratio) of about 1:10:0.1 to about 1:5,000:10.

For example, the selective PDE4 inhibitor, the ascorbic acid or the derivative thereof, and the platinum compound are administered at a mass ratio (or an effective amount ratio) of about 1:10:0.1 to about 1:5,000:10.

For example, the medicament is formulated such that the selective PDE4 inhibitor, the ascorbic acid or the derivative thereof, and the platinum compound are administered at a mass ratio (or effective amount ratio) of about to about 1:20:1, about 1:5:0.5 to about 1:20:1, about 1:10:0.1 to about 1:20:0.5, about 1:10:2 to about 1:25:0.5, about 1:10:0.5 to about 1:50:0.5, about 1:10:0.5 to about 1:100:0.5, about 1:10:0.5 to about 1:150:0.5, about 1:10:0.5 to about

1:200:0.5, about 1:10 to about 1:250:0.5, about 1:10:0.5 to about 1:300:0.5, about 1:10 to about 1:400:0.5, about 1:10:1 to about 1:450:1, about 1:10:2 to about 1:500/5, about 1:10:2 to about 1:550:2, about 1:10:0.5 to about 1:600:5, about 1:10:0.5 to about 1:650:1, about 1:10:1 to about 1:700:5, about 1:10:1 to about 1:800:2, about 1:10:0.5 to about 1:900:5, about 1:10:0.5 to about 1:1,000:1, about 1:100:1 to about 1:1500:3, about 1:10:0.1 to about 1:2,000:1, about 1:10:0.1 to about 1:3,000:2, about 1:10:1 to about 1:4,000:1, about 1:10:0.5 to about 1:5,000:1. about 1:5:0.5 to about 1:20:1, about 1:5:0.5 to about 1:25:1, about 1:50.5 to about 1:35:1, about 1:5:0.5 to about 1:50:1, about 1:5:0.5 to about 1:100:1, about 1:5:1 to about 1:150:2, about 1:5:0.5 to about 1:200:1, about 1:25:1 to about 1:50:1, about 1:25:1 to about 1:100:1, about 1:25:1 to about 1:150:2, about 1:25:1 to about 1:200:5, about 1:25:2 to about 1:1,000:2, about 1:25:0.5 to about 1:5,000:1, about 1:50:0.5 to about 1:100:1, about 1:50:0.5 to about 1:150:2, about 1:50:0.5 to about 1:200:2, about 1:50:0.5 to about 1:300:5, about 1:50:0.5 to about 1:500:5, about 1:50:0.5 to about 1:1,000:5, about 1:50:0.5 to about 1:2,000:10, about 1:50:1 to about 1:4,000:10 or about 1:50:5 to about 1:5,000:10, about 1:4500:1 to about 1:5,000:5, about 1:100:1 to about 1:2,000:5, about 1:1,000:0.5 to about 1:4,000:5, about 1:1,000:0.5 to about 1:3,000:5, about 1:5.0:1 to about 1:5,000:1, or about 1:500:1 to about 1:2,000:1.

For example, the selective PDE4 inhibitor, the ascorbic acid or the derivative thereof, and the platinum compound are administered at a mass ratio (or effective amount ratio) of about 1:1:0.1 to about 1:20:1, about 1:5:0.5 to about 1:20:1, about 1:10:0.1 to about 1:20:0.5, about 1:10:2 to about 1:25:0.5, about 1:10:0.5 to about 1:50:0.5, about 1:10:0.5 to about 1:100:0.5, about 1:10:0.5 to about 1:150:0.5, about 1:10:0.5 to about 1:200:0.5, about 1:10 to about 1:250:0.5, about 1:10:0.5 to about 1:300:0.5, about 1:10 to about 1:400:0.5, about 1:10:1 to about 1:450:1, about 1:10:2 to about 1:500:0.5, about 1:10:2 to about 1:550:2, about 1:10:0.5 to about 1:600:5, about 1:10:0.5 to about 1:650:1, about 1:10:1 to about 1:700:5, about 1:10:1 to about 1:800:2, about 1:10:0.5 to about 1:900:5, about 1:10:0.5 to about 1:1,000:1, about 1:100:1 to about 1:1500:3, about 1:10:0.1 to about 1:2,000:1, about 1:10:0.1 to about 1:3,000:2, about 1:10:1 to about 1:4,000:1, about 1:10:0.5 to about 1:5,000:1, about 1:5:0.5 to about 1:20:1, about 1:5:0.5 to about 1:25:1, about 1:50.5 to about 1:35:1, about 1:5:0.5 to about 1:50:1, about 1:5:0.5 to about 1:100:1, about 1:5:1 to about 1:150:2, about 1:5:0.5 to about 1:200:1, about 1:25:1 to about 1:50:1, about 1:25:1 to about 1:100:1, about 1:25:1 to about 1:150:2, about 1:25:1 to about 1:200:5, about 1:25:2 to about 1:1,000:2, about 1:25:0.5 to about 1:5,000:1, about 1:50:0.5 to about 1:100:1, about 1:50:0.5 to about 1:150:2, about 1:50:0.5 to about 1:200:2, about 1:50:0.5 to about 1:300:5, about 1:50:0.5 to about 1:500:5, about 1:50:0.5 to about 1:1,000:5, about 1:50:0.5 to about 1:2,000:10, about 1:50:1 to about 1:4,000:10 or about 1:50:5 to about 1:5,000:10, about 1:4500:1 to about 1:5,000:5, about 1:100:1 to about 1:2,000:5, about 1:1,000:0.5 to about 1:4,000:5, about 1:1,000:0.5 to about 1:3,000:5, about 1:500:1 to about 1:5,000:1, or about 1:500:1 to about 1:2,000:1.

For example, the medicament is formulated such that the selective PDE4 inhibitor, the ascorbic acid or the derivative thereof, and the platinum compound are administered at a mass ratio (or an effective amount ratio) of about 1:50:1 to about 1:500:1.

For example, the selective PDE4 inhibitor, the ascorbic acid or the derivative thereof, and the platinum compound are administered at a mass ratio (or an effective amount ratio) of about 1:50:1 to about 1:500:1.

For example, the effective amount ratio may comprise an effective amount mole ratio and/or an effective amount mass ratio.

For example, the administration may comprise intravenous, arterial, subcutaneous, intramuscular, intradermal, intracavitary, intratumoral, peritumoral administration, etc., for example, oral administration, application, etc. The method of administration depends on a variety of factors. For example, in order to achieve an effective concentration at the site of the tumor, the medicament may be administered in a variety of ways, for example, selective arterial infusion, intracavitary infusion, and intraperitoneal or intrapleural and intraspinal administration. For instance, in the case of local administration, for example, selective arterial, intratumoral, peritumoral injection or placement is conducted; and for example, administration is conducted in the form of intratumoral, peritumoral or intralesional slow-release.

## Selective PDE4 Inhibitor

In the present application, the selective PDE4 inhibitor may comprise substances capable of at least partially destroying or blocking the activity of one or more members of the PDE4 subfamily.

For example, the members of the PDE4 subfamily may comprise PDE4A, PDE4B, PDE4C, and/or PDE4D.

For example, the selective PDE4 inhibitor may comprise a substance that at least partially destroys or blocks the activity of PDE4A, PDE4B, PDE4C, or PDE4D.

For example, the selective PDE4 inhibitor may comprise a substance that at least partially destroys or blocks the activities of PDE4C and PDE4D. For example, the selective PDE4 inhibitor may comprise a substance that at least partially destroys or blocks the activities of PDE4B and PDE4C. For example, the selective PDE4 inhibitor may comprise a substance that at least partially destroys or blocks the activities of PDE4B and PDE4D. For example, the selective PDE4 inhibitor may comprise a substance that at least partially destroys or blocks the activities of PDE4A and PDE4B. For example, the selective PDE4 inhibitor may comprise a substance that at least partially destroys or blocks the activities

of PDE4A and PDE4C. For example, the selective PDE4 inhibitor may comprise a substance that at least partially destroys or blocks the activities of PDE4A and PDE4D.

For example, the selective PDE4 inhibitor may comprise a substance that at least partially destroys or blocks the activities of PDE4A, PDE4B and PDE4C. For example, the selective PDE4 inhibitor may comprise a substance that at least partially destroys or blocks the activities of PDE4A, PDE4C and PDE4D. For example, the selective PDE4 inhibitor may comprise a substance that at least partially destroys or blocks the activities of PDE4A, PDE4B and PDE4D. For example, the selective PDE4 inhibitor may comprise a substance that at least partially destroys or blocks the activities of PDE4B, PDE4C and PDE4D.

For example, the selective PDE4 inhibitor may comprise a substance that at least partially destroys or blocks the activities of PDE4A, PDE4B, PDE4C and PDE4D.

For example, the at least partially destroying or blocking may comprise destroying or blocking by at least 30%, 40%, 50%, 55%, 60%, 65%, 70%, 75%, 80%, 85%, 90%, or 95%.

For example, the selective PDE4 inhibitor does not substantially destroy or block the activities of other members of the PDEs family (except PDE4). For example, the substantially not destroying or blocking may comprise destroying or blocking by up to 30%, 25%, 20%, 18%, 15%, 13%, 10%, 8%, 5%, 3%, or 1%.

For example, other members of the PDEs family may include members of the PDE1 subfamily, the PDE2 subfamily, the PDE3 subfamily, the PDE5 subfamily, the PDE6 subfamily, the PDE7 subfamily, the PDE8 subfamily, the PDE9 subfamily, the PDE10 subfamily, the PDE11 subfamily, and the PDE12 subfamily.

For example, the activity may include the activity of hydrolyzing cAMP and/or cGMP. For example, the activity of hydrolyzing cAMP and/or cGMP can be determined by any method known to those skilled in the art.

For example, the selective PDE4 inhibitor may comprise apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

For example, the selective PDE4 inhibitor comprises a compound represented by Formula I or a pharmaceutically acceptable salt thereof,

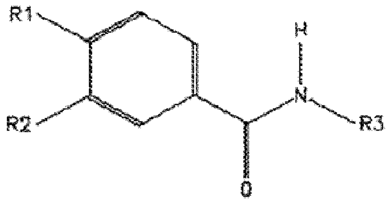 (I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and

R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,
R32 is hydrogen, hydroxy, halogen, amino, trifluoromethyl, $C_1$-$C_4$ alkyl, or $C_1$-$C_4$ alkoxy,
R33 is hydrogen, halogen, $C_1$-$C_4$ alkyl, or $C_1$-$C_4$ alkoxy,
R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl,
R36 is hydrogen or halogen, and
R37 is hydrogen or halogen.

For example, the selective PDE4 inhibitor comprises roflumilast or a derivative thereof.

For example, the derivative of the roflumilast comprises benzamide, bezafibrate and/or 4-methylbenzanilide.

For example, the compound having the structure represented by Formula I may comprise the compound described in the Patent Application No. US5712298A, which is incorporated herein by reference.

For example, in Forumula I: R1 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine,

R2 is $C_1$-$C_4$ alkoxy fully or partially substituted by fluorine, and
R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, C1-C4 alkoxy, $C_1$-$C_4$ alkoxycarbonyl, $C_1$-$C_4$ alkylcarbonyl, $C_1$-$C_4$ alkylcarbonyloxy, amino, mono-or di-$C_1$-$C_4$ alkylamino, or $C_1$-$C_4$ alkylcarbonylamino, R32 is hydrogen, hydroxy, halogen, amino, trifluoromethyl, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl, R36 is hydrogen or halogen, and R37 is hydrogen or halogen.

For example, in Formula I: R1 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, R2 is methoxy fully or partially substituted by fluorine, and R3 is phenyl, pyridyl, phenyl substituted by R31, R32 and R33, or pyridyl substituted by R34, R35, R36 and R37, wherein: R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono-or di-$C_1$-$C_4$ alkylamino, or $C_1$-$C_4$ alkylcarbonylamino, R32 is hydrogen, hydroxy, halogen, amino, trifluoromethyl, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amno, or $C_1$-$C_4$ alkyl, R36 is hydrogen or halogen, and R37 is hydrogen or halogen.

For example, in Formula I: R1 is $C_3$-$C_7$ cycloalkoxy, $C_3$-$C_7$ cycloalkylmethoxy, or benzyloxy, R2 is $C_1$-$C_4$ alkoxy fully or partially substituted by fluorine, and R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono-or di-$C_1$-$C_4$ alkylamino, or $C_1$-$C_4$ alkylcarbonylamino, R32 is hydrogen, hydroxy, halogen, amino, trifluoromethyl, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl, R36 is hydrogen or halogen, and R37 is hydrogen or halogen.

For example, in Formula I: R1 is $C_1$-$C_4$ alkoxy fully or partially substituted by fluorine, R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$-alkylcarbonyloxy, amino, mono-or di-$C_1$-$C_4$ alkylamino, or $C_1$-$C_4$ alkylcarbonylamino, R32 is hydrogen, hydroxy, halogen, amino, trifluoromethyl, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or 1-4C-alkyl, R36 is hydrogen or halogen, and R37 is hydrogen or halogen.

For example, in Formula I: R1 is $C_1$-$C_4$ alkoxy fully or partially substituted by fluorine, R2 is $C_3$-$C_7$ cycloalkylmethoxy or benzyloxy, and R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono-or di-$C_1$-$C_4$ alkylamino, or $C_1$-$C_4$ alkylcarbonylamino, R32 is hydrogen, hydroxy, halogen, amino, trifluoromethyl, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl, R36 is hydrogen or halogen, and R37 is hydrogen or halogen.

For example, in Formula I: R1 is $C_1$-$C_4$ alkoxy, $C_3$-$C_5$ cycloalkoxy, $C_3$-$C_5$ cycloalkylmethoxy, benzyloxy, or $C_1$-$C_4$ alkoxy fully or partially substituted by fluorine, R2 is $C_1$-$C_4$ alkoxy fully or partially substituted by fluorine, and R3 is phenyl, pyridyl, phenyl substituted by R31, R32 and R33, or pyridyl substituted by R34, R35, R36 and R37, wherein: R31 is halogen, cyano, carboxy, $C_1$-$C_4$ alkyl, $C_1$-$C_4$ alkoxy, or $C_1$-$C_4$ alkoxycarbonyl, R32 is hydrogen, halogen, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is halogen or $C_1$-$C_4$ alkyl, R35 is hydrogen or halogen, R36 is hydrogen or halogen, and R37 is hydrogen or halogen.

For example, in Formula I: R1 is $C_1$-$C_4$ alkoxy, $C_3$-$C_5$ cycloalkoxy, $C_3$-$C_5$ cycloalkylmethoxy, benzyloxy, or $C_1$-$C_4$ alkoxy fully or partially substituted by fluorine, R2 is methoxy fully or partially substituted by fluorine, and R3 is phenyl, pyridyl, phenyl substituted by R31, R32 and R33, or pyridyl substituted by R34, R35, R36 and R37, wherein: R31 is halogen, cyano, carboxy, $C_1$-$C_4$ alkyl, $C_1$-$C_4$ alkoxy, or $C_1$-$C_4$ alkoxycarbonyl, R32 is hydrogen, halogen, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is halogen or $C_1$-$C_4$ alkyl, R35 is hydrogen or halogen, R36 is hydrogen or halogen, and R37 is hydrogen or halogen.

For example, in Formula I: R1 is $C_3$-$C_5$ cycloalkoxy, $C_3$-$C_5$ cycloalkylmethoxy, or benzyloxy, R2 is $C_1$-$C_4$ alkoxy fully or partially substituted by fluorine, and R3 is phenyl, pyridyl, phenyl substituted by R31, R32 and R33, or pyridyl substituted by R34, R35, R36 and R37, wherein:

R31 is halogen, cyano, carboxy, $C_1$-$C_4$ alkyl, $C_1$-$C_4$ alkoxy, or $C_1$-$C_4$ alkoxycarbonyl, R32 is hydrogen, halogen, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is halogen or $C_1$-$C_4$ alkyl, R35 is hydrogen or halogen, R36 is hydrogen or halogen, and R37 is hydrogen or halogen.

For example, in Formula I: R1 is $C_1$-$C_4$ alkoxy fully or partially substituted by fluorine, R2 is $C_1$-$C_4$ alkoxy, $C_3$-$C_5$ cycloalkoxy, $C_3$-$C_5$ cycloalkylmethoxy, benzyloxy, or $C_1$-$C_4$ alkoxy fully or partially substituted by fluorine, and R3 is phenyl, pyridyl, phenyl substituted by R31, R32 and R33, or pyridyl substituted by R34, R35, R36 and R37, wherein: R31 is halogen, cyano, carboxy, $C_1$-$C_4$ alkyl, $C_1$-$C_4$ alkoxy, or $C_1$-$C_4$ alkoxycarbonyl, R32 is hydrogen, halogen, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is halogen or $C_1$-$C_4$ alkyl, R35 is hydrogen or halogen, R36 is hydrogen or halogen, and R37 is hydrogen or halogen.

For example, in Formula I: R1 is $C_1$-$C_4$ alkoxy fully or partially substituted by fluorine, R2 is $C_3$-$C_5$ cycloalkylmethoxy or benzyloxy, and R3 is phenyl, pyridyl, phenyl substituted by R31, R32 and R33, or pyridyl substituted by R34, R35, R36 and R37, wherein: R31 is halogen, cyano, carboxy, $C_1$-$C_4$ alkyl, $C_1$-$C_4$ alkoxy, or $C_1$-$C_4$ alkoxycarbonyl, R32 is hydrogen, halogen, $C_1$-$C_4$ alkyl, or $C_1$-$C_4$ alkoxy, R33 is hydrogen, halogen, $C_1$-$C_4$ alkyl, or $C_1$-$C_4$ alkoxy, R34 is halogen or $C_1$-$C_4$ alkyl, R35 is hydrogen or halogen, R36 is hydrogen or halogen, and R37 is hydrogen or halogen.

For example, in Formula I: R1 is $C_1$-$C_4$ alkoxy, $C_1$-$C_4$ cycloalkoxy, $C_1$-$C_4$ cycloalkylmethoxy, benzyloxy, or $C_1$-$C_4$ alkoxy fully or partially substituted by fluorine, R2 is $C_1$-$C_4$ alkoxy fully or partially substituted by fluorine, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is $C_1$-$C_4$ alkoxy, $C_3$-$C_5$ cycloalkoxy, $C_3$-$C_5$ cycloalkylmethoxy, benzyloxy, or $C_1$-$C_4$ alkoxy fully or partially substituted by fluorine, R2 is methoxy fully or partially substituted by fluorine, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3, dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2, dichloropyridine-3-yl.

For example, in Formula I: R1 is $C_3$-$C_5$ cycloalkoxy, $C_3$-$C_5$ cycloalkylmethoxy, or benzyloxy, R2 is $C_1$-$C_4$ alkoxy fully or partially substituted by fluorine, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is $C_1$-$C_4$ alkoxy fully or partially substituted by fluorine, R2 is $C_1$-$C_4$ alkoxy, $C_3$-$C_5$ cycloalkoxy, $C_3$-$C_5$ cycloalkylmethoxy, benzyloxy, or $C_1$-$C_4$ alkoxy fully or partially substituted by fluorine, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is $C_1$-$C_4$ alkoxy fully or partially substituted by fluorine, R2 is $C_3$-$C_5$ cycloalkylmethoxy or benzyloxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is difluoromethoxy, R2 is methoxy, ethoxy, isopropoxy, isobutoxy, cyclopentyloxy, cyclopropylmethoxy, cyclobutylmethoxy, difluoromethoxy, or 2,2,2-trifluoroethoxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is difluoromethoxy, R2 is methoxy, cyclopropylmethoxy, cyclobutylmethoxy, or difluoromethoxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is methoxy, n-propoxy, n-butoxy, cyclopropylmethoxy, or 2,2,2-trifluoroethoxy, R2 is difluoromethoxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, in Formula I: R1 is difluoromethoxy, R2 is cyclopropylmethoxy, and R3 is 2-bromophenyl, 2,6-dichloro-4-ethoxycarbonylphenyl, 2,6-dimethoxyphenyl, 4-cyano-2-fluorophenyl, 2,4,6-trifluorophenyl, 2-chloro-6-methylphenyl, 2,6-dimethylphenyl, 2,6-difluorophenyl, 2,6-dichlorophenyl, 3,5-dichloropyridine-4-yl, 3-methylpyridine-2-yl, 2-chloropyridine-3-yl, 3,5-dibromopyridine-2-yl, 2,3,5,6-tetrafluoropyridine-4-yl, 3-chloro-2,5,6-trifluoropyridine-4-yl, 3,5-dichloro-2,6-difluoropyridine-4-yl, or 2,6-dichloropyridine-3-yl.

For example, the compound having the structure represented by Formula I may comprise: N-(3,5-dichloropyridine-4-yl)-4-difluoromethoxy-3-methoxybenzamide (with a melting point of 170°C), N-(3,5-dichloropyridine-4-yl)-3, 4-bis-difluoromethoxy benzamide (melting point: 134°C), N-(3,5-dichloropyridine-4-yl)-3-cyclobutylmethoxy-4-difluoromethoxy benzamide (melting point: 155°C), N-(3,5-dichloropyridine-4-yl)-3-cyclopentyloxy-4-difluoromethoxy benzamide (melting point: 128.5-129°C), N-(3,5-dichloropyridine-4-yl)-3-cyclopropylmethoxy-4-difluoromethoxy benzamide (melting point: 158°C), N-(3,5-dichloro-2,6-difluoropyridine-4-yl)-3-difluoromethoxy-4-methoxybenzamide (melting point: 218°C), N-(2,6-dichlorophenyl)-3-difluoromethoxy-4-methoxybenzamide (melting point: 164°C), N-(2,6-dimethylphenyl)-3-difluoromethoxy-4-methoxybenzamide (melting point: 164°C), N-(2-chloropyridine-3-yl)-3-difluoromethoxy-4-methoxybenzamide (melting point: 165°C), N-(3,5-dibromopyridine-2-yl)-3-difluoromethoxy-4-methoxybenzamide (melting point: 143°C), N-(3,5-dichloropyridine-4-yl)-3-difluoromethoxy-4-methoxybenzamide (melting point: 178°C), N-(3,5-dichloropyridine-4-yl)-3-difluoromethoxy-4-propoxybenzamide (melting point: 159°C), N-(3,5-dichloropyridine-4-yl)-3-ethoxy-4-difluoromethoxy benzamide (melting point: 134°C), N-(3,5-dichloropyridine-4-yl) benzyloxy-3-difluoromethoxy benzamide (melting point: 152°C), N-(3,5-dichloropyridine-4-yl)-4-butoxy-3-difluoromethoxy benzamide (melting point: 146°C), N-(3,5-dichloropyridine-4-yl)-4-cyclopropylmethoxy-3-difluoromethoxy benzamide (melting point: 159°C), N-(3,5-dichloropyridine-4-yl)-4-difluoromethoxy-3-(1-methylethoxy)benzamide (melting point: 99.5°C), N-(3,5-dichloropyridine-4-yl)-4-difluoromethoxy-3-(2,2,2-trifluoroethoxy)-benzamide (melting point: 147°C), N-(3,5-dichloropyridine-4-yl)-4-difluoromethoxy-3-(2-methylpropoxy)benzamide (melting point: 153°C), N-(2,3,5,6-tetrafluoropyridine-4-yl)-4-difluoromethoxy-3-methoxybenzamide (melting point: 146°C), N-(2,4,6-trifluorophenyl)-4-difluoromethoxy-3-methoxybenzamide (melting point: 145°C), N-(2,6-dichloro-4-ethoxycarbonylphenyl)-4-difluoromethoxy-3-methoxybenzamide (melting point: 176°C), N-(2,6-dichlorophenyl)-4-difluoromethoxy-3-methoxybenzamide (melting point: 186°C), N-(2,6-difluorophenyl)-4-difluoromethoxy-3-methoxybenzamide (melting point: 139°C), N-(2,6-dimethylphenyl)-4-difluoromethoxy-3-methoxybenzamide (melting point: 143°C), N-(2-bromophenyl)-4-difluoromethoxy-3-methoxybenzamide (melting point: 121°C), N-(2-chloro-6-methylphenyl)-4-difluoromethoxy-3-methoxybenzamide (melting point: 144°C), N-(2-chloropyridine-3-yl)-4-difluoromethoxy-3-methoxybenzamide (melting point: 137.5°C), N-(2-methylphenyl)-4-difluoromethoxy-3-methoxybenzamide (melting point: 125°C), N-(3,5-dibromopyridine-2-yl)-4-difluoromethoxy-3-methoxybenzamide (melting point: 141°C), N-(3,5-dichloro-2,6-difluoropyridine-4-yl)-4-difluoromethoxy-3-methoxybenzamide (melting point: 174°C), N-(3-chloro-2,5,6-trifluoropyridine-4-yl)-4-difluoromethoxy-3-methoxybenzamide (melting point: 141°C), N-(3-methylpyridine2-yl)-4-difluoromethoxy-3-methoxybenzamide (melting point: 96°C).

For example, depending on the substitution reaction, suitable salts of the compound of Formula I may be all acid addition salts or all salts formed from bases. Of particular mention are salts prepared from pharmacologically tolerable inorganic and organic acids and bases that are commonly used in pharmacy. In the case of pharmacologically intolerable salts, when the compound of the present invention is prepared, for example, such salts must be first precipitated as an intermediate product on an industrial scale, and then converted into pharmacologically tolerable salts by using a method familiar to professionals. In one aspect, the suitable salts are water-soluble and non-water-soluble acid addition salts formed with acids, and acids suitable for this purpose comprise, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, pamoic acid, stearic acid, toluenesulfonic acid, methanesulfonic acid or 3-hydroxy-2-naphthoic acid. Here, during the preparation of a salt, the acid used may be a monobasic or polybasic acid as required, and depending on what kind of salt is needed, a feeding ratio may be quantified by mole, or feeding may be conducted by deviating from this quantified ratio.

For example, the salt may also be formed with a base. As examples of basic salts mentioned, there are, for example lithium, sodium, potassium, calcium, aluminum, magnesium, titanium, ammonium, methyl meglumine- or guanidinium salts. Here, during the preparation of a salt, the base may be quantified by mole, or feeding may be conducted by deviating from this quantified ratio.

## Platinum Compound

In the present application, the platinum compound may comprise a class of platinum-containing substance, for example, cisplatin (DDP), carboplatin, heptanoplatin, denatoplatin, enloplatin, sulfatodaiaminocyclohxane platinum (cycloethylenediamine platinum sulfate), cis-spiroplatin, dexormaplatin, iproplatin, lobaplatin, miboplatin, nedaplatin, ormaplatin, oxaliplatin, sebriplatin, spiroplatin, and/or zeniplatin.

## Ascorbic Acid and Derivatives Thereof

In the present application, the ascorbic acid and the derivative thereof comprises members selected from ascorbic acid (vitamin C), the derivative of ascorbic acid, and a combination thereof.

For example, the derivative of ascorbic acid may further comprise ascorbate or a salt of the derivative of the ascorbic acid.

For example, the derivative of ascorbic acid may further comprise dehydroascorbate or a salt thereof.

The ascorbic acid is sensitive to the influence of environmental parameters (for example, light, heat, and oxygen) due to its α-ketolactone structure. It may be unstable in water or other aqueous solutions. The derivative of ascorbic acid with higher stability than the parent compound is prepared by means of chemically stabilizing the ascorbic acid molecule (for example, see US Patent Nos. US5137723A and US5078989A), the disclosures of which are incorporated into the present application by reference

For example, the derivative of the ascorbic acid may be an ascorbic acid analog. Typical derivatives of the ascorbic acid comprise a compound in which at least one of the hydroxyl groups (for example, 2-OH, 3-OH, 5-OH, and 6-OH) of the ascorbic acid molecule is derivatized by a modified group (see, for example, Ando et al., U.S. Patent No. US5078989A). For example, one or more of the hydroxyl groups may be substituted by another structural moiety. For example, the ascorbic acid and the derivative thereof comprise ascorbic acid and at least one derivative of the ascorbic acid.

For example, the derivative of the ascorbic acid may comprise free 2-OH and free 3-OH.

For example, the derivative of the ascorbic acid comprises ester of the ascorbic acid, which is derived from at least one of 5-OH and 6-OH.

For example, the derivative of the ascorbic acid comprises esters, for example, 6-O-octanoyl-ascorbic acid, 6-O-dodecanoyl-ascorbic acid, 6-O-tetradecanoyl-ascorbic acid, 6-O-octadecanoyl-ascorbic acid, 6-O-dodecanedioyl-ascorbic acid, 6-O-docosanedioyl-ascorbic acid, 6-O-thapsoyl-ascorbic acid, 6-O-decanedioyl-ascorbic acid, and 6-O-adipyl-ascorbic acid.

For example, the derivative of the ascorbic acid may also comprise esters in which the lipophilic structural moiety of the molecule is a mono- or poly-unsaturated fatty acid. For example, the unsaturated fatty acids may comprise essential fatty acids related to health, such as ω-3(α-linolenic acid), ω-6, or ω-9 fatty acids. For example, they may further comprise esters containing amino acid residues.

For example, the derivative of the ascorbic acid may also comprise a 2-O-alkyl or 3-O-alkyl derivative of the ascorbic acid. The 3-O-alkyl-ascorbic acid was reported by Nihro et al. in Chem. Pharm. Bull. 1991, 39: 1731-1735, which is incorporated into the present application by reference.

For example, the derivative of the ascorbic acid may comprise glycosides of the ascorbic acid, for example, ascorbyl 1-glycoside, ascorbyl 2-glycoside, ascorbyl 3-glycoside, ascorbyl 5-glycoside, and ascorbyl 6-glycoside.

For example, the derivative of the ascorbic acid may comprise 2-O-(α-D-glucopyranosyl)-ascorbic acid (see, for example, U.S. Patent No. US5137723A) and 2-O-(β-D-glucopyranosyl)-ascorbic acid (see, for example, U.S. Patent Application No. US20050113312A1). The above documents are incorporated into the present application by reference.

For example, the derivative of the ascorbic acid may comprise bifunctionalized derivatives of the ascorbic acid, such as, for example, 6-O-acyl-2-O-(α-D-glucopyranosyl) ascorbic acid (see, for example, Yamamoto et al., J Med. Chem. 2002, 45(2): 462-468). This document is incorporated into the present application by reference.

For example, the derivative of the ascorbic acid may comprise phosphates of the ascorbic acid. For example, the phosphates of the ascorbic acid comprise an alkali metal salt, an alkaline earth metal salt, or a transition metal salt. For example, magnesium ascorbyl phosphate, sodium ascorbyl phosphate (for example, sodium ascorbyl-2-monophosphate), calcium ascorbyl phosphate, potassium ascorbyl phosphate, and a mixture salt (such as sodium magnesium ascorbyl phosphate, sodium calcium ascorbyl phosphate, aminopropyl ascorbyl phosphate) may be included.

For example, the phosphates of the ascorbic acid can exist as hydrates, among which dihydrates are common. Typical dihydrates can be purchased, for example, from DSM under the product name STAY-C50.

In the present application, the derivative of the ascorbic acid comprises pharmaceutically acceptable ascorbate.

For example, the pharmaceutically acceptable ascorbate comprises its alkali metal salts (such as sodium salt and potassium salt), alkaline earth metal salts (such as calcium salt and magnesium salt), basic amino acid salts (such as arginine), and organic amine salts (such as triethanolamine).

For example, the ascorbate or the salt of the derivative of ascorbic acid may be an edible (for example, pharmaceutically acceptable) salt (such as calcium, sodium, magnesium, potassium, and zinc salts), and a mixture salt of the ascorbic acid or the derivative of the ascorbic acid.

For example, the pharmaceutically acceptable ascorbate comprises sodium ascorbate.

Other recognized derivatives of the ascorbic acid are also used for the purpose of the present application.

## Carriers and Formulations

The ascorbic acid or the derivative thereof of the present application, the selective PDE4 inhibitor of the present application, and the platinum compound of the present application may each be an independent formulation (namely, the first formulation, the second formulation, and the third formulation), or may also be prepared into an appropriate formulation in the form of a composition, for example, a composition of the ascorbic acid or the derivative thereof of the present application and the selective PDE4 inhibitor of the present application, for example, a composition of the ascorbic acid or the derivative thereof of the present application and the platinum compound of the present application, for example, a composition of the selective PDE4 inhibitor of the present application and the platinum compound of the present application, and for example, a composition of the ascorbic acid or the derivative thereof of the present application, the selective PDE4 inhibitor of the present application, and the platinum compound of the present application. The formulation prepared either independently or in the form of a composition can be combined with any suitable carrier to achieve the desired formulation type.

For example, the dosage form of the first formulation, the second formulation, the third formulation, or the pharmaceutical composition may comprise tablets, capsules, granules, powders, syrups, suspensions, suppositories, ointments, creams, gels, patches, inhalants, injections, etc. These formulations can be prepared according to a conventional method.

For example, in the case of a liquid formulation, it can be in the form of being dissolved or suspended in water or other suitable solvents when in use.

For example, the tablets and granules can be coated by a known method.

For example, in the case of an injection, it can be prepared by dissolving active ingredients (comprising the ascorbic acid or the derivative thereof of the present application, the selective PDE4 inhibitor of the present application, and the platinum compound of the present application) in water, or as required, in normal saline or a glucose solution. For example, a buffering agent and a preservative can also be added.

For example, it can also be provided in any formulation form for oral administration or paraoral administration. For example, it can be prepared into a formulation for oral administration in the form of granules, fine granules, powders, hard capsules, soft capsules, syrups, emulsions, suspensions or liquids.

For example, a formulation for paraoral administration in the form of injections, drops, transdermal absorbers, transmucosal absorbers, nasal drop, inhalants, suppositories, etc. for intravenous, intramuscular, intradermal, intracavitary, intratumoral, peritumoral, or subcutaneous administration, etc. may also be used.

For example, the injections, drips, etc. can be prepared into a powdered dosage form such as a freeze-dried form, and dissolved in a suitable aqueous medium such as normal saline for use.

For example, a sustained-release formulation covered with a polymer or the like may also be directly administered to organs and tissues such as the brain, blood, and lymph, or into a tumor. For example, the sustained-release formulation may comprise a sustained-release pump, a sustained-release capsule, a sustained-release agent, an implant, or a sustained-release implant.

In the sense of the type of formulation additives (carriers) used in the manufacture of a pharmaceutical formulation, the ratio of formulation additives (carriers) to active ingredients, or the manufacturing method for the formulation, those skilled in the art can make an appropriate choice according to the form of the formulation. For example, as the additives (carriers) for the formulation, inorganic or organic substances, or solid or liquid substances can be used. For example, blending can be performed with respect to the weight of the active ingredients in the range of 1% to 90% by weight. For example, the carriers may comprise lactose, glucose, mannitol, dextrin, cyclodextrin, starch, sucrose, magnesium aluminum metasilicate, synthetic aluminum silicate, sodium carboxymethyl cellulose, hydroxypropyl starch, calcium carboxymethyl cellulose, ion exchange resin, methyl cellulose, gelatin, arabic gum, hydroxypropyl cellulose, hydroxypropyl methyl cellulose, polyvinylpyrrolidone, polyvinyl alcohol, light anhydrous silicic acid, magnesium stearate, talc, tragacanth, bentonite, propolis, titanium oxide, sorbitan fatty acid ester, sodium lauryl sulfate, glycerin, fatty glyceride, purified lanolin, gelatin glycerin, polysorbate, polyethylene glycol, vegetable oil, wax, liquid paraffin, white petrolatum, fluorocarbon, non-ionic surfactant, propylene glycol, water, etc.

For example, when solid formulations for oral administration are produced, active ingredients (comprising the ascorbic acid or the derivative thereof of the present application, the selective PDE4 inhibitor of the present application, and the platinum compounds of the present application) can be mixed with a carrier ingredient (such as lactose, starch, crystalline cellulose, calcium lactate, or anhydrous silicic acid) to prepare powders. For example, a binder (such as sucrose, hydroxypropyl cellulose, or polyvinylpyrrolidone) and a disintegrating agent (such as carboxymethyl cellulose or carboxymethyl cellulose calcium) can also be added as required, and then subjected to wet or dry granulation to prepare granules. For example, when tablets are produced, the powders and/or granules as described can be directly tableted. For example, lubricants such as magnesium stearate and talc can also be added for tableting. For example, the granules or tablets as described can also be coated with hydroxypropyl methylcellulose phthalate, a methacrylate-methyl methacrylate polymer and other enteric-coated agent matrixes to produce enteric-coated agents. For example,

ethyl cellulose, carnauba wax, hydrogenated oil, etc. can also be used for coating to produce long-acting formulations. For example, when capsules are produced, powders or granules can be filled in hard capsules. For example, the active ingredients as described can also be covered with a gelatin film directly or after being dissolved in glycerin, polyethylene glycol, sesame oil, olive oil, etc., in order to produce soft capsules.

For example, when injections are produced, the active ingredients can be dissolved together with a pH adjusting agent (such as hydrochloric acid, sodium hydroxide, lactose, lactic acid, sodium, sodium hydrogen phosphate, and sodium dihydrogen phosphate) and an isotonic agent (such as sodium chloride, or glucose) as required in distilled water for injection. For example, they can be further sterilely filtered and filled in an ampoule. For example, mannitol, dextrin, cyclodextrin, gelatin, etc. can be further added and lyophilized in vacuum to prepare an injection that is dissolved for use. For example, lecithin, polysorbate 80, polyoxyethylene hydrogenated castor oil, etc. can also be added to the active ingredients and emulsified in water to prepare an emulsion for injection.

For example, when agents for rectal administration are produced, the active ingredients can be humidified and dissolved together with a suppository matrix, such as cocoa butter, fatty acid triglyceride, fatty acid diglyceride, fatty acid monoglyceride, and polyethylene glycol, and then are allowed to flow into a mold for cooling. For example, the active ingredients may also be dissolved in polyethylene glycol, soybean oil, etc., and then coated with a gelatin film.

For example, when external formulations for skin are produced, the active ingredients can be added to white petrolatum, beeswax, liquid paraffin, polyethylene glycol, etc., and humidified and kneaded as required to produce ointments. For example, it can also be kneaded with a binder such as rosin and an alkyl acrylate polymer and then spread on a polyalkyl-based non-woven fabric to produce a bandage-like formulation.

For example, it can also be used as a sustained-release formulation such as an implant or a delivery system encapsulated in a microcapsule, which can be prepared using a carrier that can prevent immediate removal in vivo. For example, biodegradable and biocompatible polymers such as ethylene-vinyl acetate, polyanhydride, polyglycolic acid, collagen, polyorthoester, and polylactic acid can be used. These materials can be readily prepared by those skilled in the art. For example, the suspension of liposome can also be used as a pharmaceutically acceptable carrier. The liposome may comprise a lipid composition containing phosphatidylcholine, cholesterol, and PEG-derivatized phosphatidyl ethanolamine (PEG-PE), and the lipid composition can be prepared in a manner of making the size suitable for use and by a filter having a suitable pore size, and purified by reverse phase evaporation.

In the present application, the dosage and the number of administrations can be appropriately selected according to conditions such as the purpose of disease progress prevention and/or treatment for a treatment object (subject), the type of disease, the weight of patient, and the age. For example, oral administration may be conducted once or several times a day, or may be administered every a few days. In the case of the injection, continuous or intermittent administration can be conducted.

Not wishing to be bound by any particular theory, the following examples are merely to illustrate the pharmaceutical combination, use methods, uses, etc. according to the present application, and are not intended to limit the scope of the present invention.

## Examples

C57 mice used in the present application were purchased from Vital River Laboratory Animal Technology Co., Ltd.; MC38 cells were purchased from ATCC; roflumilast was purchased from Aladdin with Item No.: R126602; oxaliplatin was purchased from Aladdin with Item No.: 0124003; and the sodium ascorbate was purchased from Aladdin, with Item No.: S105024. Oxaliplatin and roflumilast were used by taking DMSO as the solvent, and the sodium ascorbate was used with PBS as the solvent.

Data processing: all data were expressed as mean ± standard deviation (Mean ± SD), and GraphPad Prism 7.0 software was used for one-way analysis of variance (One-way ANOVA). When $P < 0.05$, it indicates a statistically significant difference. * means $P < 0.05$, ** means $P < 0.01$, and *** means $P < 0.001$.

The nature of good drug interaction was evaluated using the coefficient of drug in interaction (CDI). A control group, a drug group A, a drug group B, and a combined drug group AB were provided to detect the cell activity in the groups A, B, and AB.

CDI = cell activity of group AB/cell activity of group A * cell activity of group B. If CDI < 1, it proves a synergistic nature of good drug interaction; if CDI < 0.7, it indicates a very significant synergistic good drug interaction; if CDI = 1, it indicates an additive nature of good drug interaction; and if CDI > 1, it indicates an antagonistic nature of good drug interaction.

## Example 1 Inhibitory effect of roflumilast and sodium ascorbate against colon cancer

(1) Tumor formation in mice. C57 female mice aged 6-8 weeks were selected, and MC38 cells were cultured in vitro to the logarithmic phase, and then collected and resuspended in the PBS buffer. The collected cells were injected into the mammary glands of mice by subcutaneous injection, and each mouse was injected with $5\times10^5$ MC38 cells to establish an in situ tumor model.

(2) After tumor formation, the mice were divided into four experimental groups, namely, a blank control, a roflumilast group, a sodium ascorbate group, and a roflumilast + sodium ascorbate group. The mice in the roflumilast + sodium ascorbate group were intraperitoneally injected with roflumilast at 4 mg/kg and intravenously injected with sodium ascorbate at 1.5 g/kg every three days, for 5 administrations. At the same time, the roflumilast group and the sodium ascorbate group were separately injected with roflumilast and sodium ascorbate at the same dose in the same way; and normal saline was injected at the same dose in the blank control. The day of the first injection was taken as Day 1, and then the mice were tested and recorded in terms of body weight and tumor size: tumor volume ($mm^3$) = length (L) $\times$ width (W) $^2/2$. Luciferin fluorescence imaging was conducted on tumors. That is, a 1 mg/mL D-luciferin potassium aqueous solution was formulated; each mouse was intraperitoneally injected with 100 $\mu$L of the D-luciferin potassium aqueous solution, and 10 minutes after injection, was subjected to luciferin fluorescence imaging with a small animal in vivo imager (Model: IVIS Lumina XRMS Series III).

The results were shown in FIG. 1 to FIG. 4. FIG. 1 showed the results of luciferin fluorescence imaging of respective groups on Day 18, and FIG. 2 was a statistical graph of the results of luciferin fluorescence imaging. Both FIG. 1 and FIG. 2 indicated that the combinational group of roflumilast and sodium ascorbate significantly suppressed tumor growth as compared with the blank control, the roflumilast group, and the sodium ascorbate group. FIG. 3 was a statistical graph of tumor volume changes over time. It showed that the combinational group of roflumilast and sodium ascorbate significantly suppressed tumor growth as compared with the blank control, the roflumilast group, and the sodium ascorbate group, and on Day 18, the significant difference analysis between the combinational group and the single-drug group of roflumilast showed $P < 0.001$. FIG. 4 showed that, compared with the control, roflumilast and sodium ascorbate, either in the single-drug group nor the combinational group, had no effect on the body weight of mice, exhibiting good safety.

**Example 2 Strengthening of anti-tumor effect of oxaliplatin by roflumilast and sodium ascorbate**

The effect of roflumilast and sodium ascorbate on the anti-tumor effect of oxaliplatin was verified according to the method of Example 1. The mice were divided into five experimental groups: a blank control, a roflumilast group, a sodium ascorbate group, an oxaliplatin group, a roflumilast + sodium ascorbate + oxaliplatin group. The mice in the roflumilast + sodium ascorbate + oxaliplatin group were intraperitoneally injected with roflumilast at 4 mg/kg and oxaliplatin at 4 mg/kg and intravenously injected with sodium ascorbate at 1.5 g/kg every three days, for 6 administrations. At the same time, the roflumilast group, the sodium ascorbate group, and the oxaliplatin group were separately injected with roflumilast, sodium ascorbate, and oxaliplatin at the same dose in the same way; and normal saline was injected at the same dose in the blank control. The day of the first injection was taken as Day 0, and then the mice were tested and recorded in terms of body weight and tumor size: tumor volume ($mm^3$) = length (L) $\times$ width (W) $^2/2$.

The results were shown in FIG. 5 and FIG. 6, indicating that roflumilast and sodium ascorbate were capable of significantly improving the anti-tumor effect of oxaliplatin, and had no effect on the body weight of mice, exhibiting good safety. FIG. 5 showed that on Day 21, the significant difference analysis between the combinational group of roflumilast + sodium ascorbate + oxaliplatin and the single-drug group of oxaliplatin showed $P < 0.001$.

**Example 3 Inhibitory effect of selective PDE4 inhibitor combined with sodium ascorbate against colon cancer cells/melanoma cells**

1.1 Cell lines and cell culture

MC38 human colon cancer cells, HCT116 human colon cancer cells, and A375 cells were subcultured using a RPMI-1640 medium containing 10% fetal bovine serum FBS in an incubator at 37°C in the presence of 5% carbon dioxide.

1.2 Cell viability test after single-drug administration

(1) After the cells spread on the 10 cm culture plate grew to a density of about 90%, the medium was removed; the adherent cells were washed once with the PBS buffer; 1 mL of trypsin was added, mixed, and discarded immediately;

the cells were digested in an incubator for about 5 minutes; then, about 10 ml of fresh medium was added; and 10 μL were pipetted for counting.

(2) After counting, the cells were diluted to $1*10^5$ cells per mL, and the diluted cells were spread into a 96-well plate at 100 uL per well, i.e., about 10,000 cells per well.

(3) After the cells spread in the 96-well plate were cultured for 24 hours, the selective PDE4 inhibitors of different concentration gradients, namely, benzanilide, 4-methylbenzanilide, apremilast, crisaborole, and drotaverine, were added to the cells.

(4) 24 hours after the addition, 10 μL of CCK-8 solution was added; the cells were incubated in the incubator for 1-2 hours; and the absorbance value (OD value) of each well was measured at 490 nm using a microplate reader, and the results were recorded. The relative cell activity was calculated as follows:

cell activity = (absorbance value of experimental group - absorbance value of blank group)/(absorbance value of control group - absorbance value of blank group) × 100%.

Here, the experimental group was a cell group added with a drug of a different concentration; the control group was a cell group added only with a solvent; and the blank group was a group to which cells were not added and a solvent was only added. The results of cell activity were obtained from the above formula, and the cell inhibition rate of the drug = 100% - cell viability. The cell viability of the control group obtained according to this formula was 100%.

1.3 Cell counting after drug combination

(1) After the cells spread on the 10 cm culture plate grew to a density of about 90%, the medium was removed; the adherent cells were washed once with the PBS buffer; 1 mL of trypsin was added, mixed, and discarded immediately; the cells were digested in an incubator for about 5 minutes; then, about 10 ml of fresh medium was added; and 10 μL were pipetted for counting.

(2) 10 μL of the mixed cells were pipetted, blended with 10 μL of trypan blue solution, and counted.

(3) After counting, the cells were diluted to $1*10^5$ cells per mL; and the diluted cells were well mixed and pipetted into a 24-well plate at 500 uL/well, i.e., about 50,000 cells per well.

(4) After the cells spread on the 24-well plate were cultured for about 24 hours, drugs were added to respective experimental groups as follows: 1) a single-drug group of selective PDE4 inhibitor, 2) a sodium ascorbate group, 3) a combinational group of selective PDE4 inhibitor and sodium ascorbate, in which the concentration of each drug was the same as that in the corresponding single-drug group, and 4) a control group, which was a cell group in which a corresponding solvent having the same volume as that in the single-drug group of selective PDE4 inhibitor was only added. The selective PDE4 inhibitors included: benzanilide, 4-methylbenzanilide, apremilast, crisaborole, and drotaverine, respectively.

(5) 24 hours after the drugs were added, the culture medium was discarded, and about 150 μL of pancreatin was added and then discarded, allowing digestion to last for about 5 minutes.

(6) After digestion, the same volume of PBS was added to each well and mixed well; then, a trypan blue solution was added at 10 μL/well and mixed well; and 10 μL of the mixed solution was pipetted for counting. The cell activity of each group was calculated according to "cell activity = the number of cells in the experimental group/the number of cells in the control group × 100%".

1.4. Data processing

The data were all expressed as mean ± standard deviation (Mean±SD), and GraphPad Prism 7.0 software was used forone-way analysis of variance (One-way ANOVA). When $P < 0.05$, it indicates a statistically significant difference. * means $P < 0.05$, ** means $P < 0.01$, and *** means $P < 0.001$.

The nature of good drug interaction was evaluated using the coefficient of drug in interaction (CDI). A control group, a drug group A, a drug group B, and a combined drug group AB were provided to detect the cell activity in the groups A, B, and AB.

CDI = cell activity of group AB/cell activity of group A * cell activity of group B. If CDI < 1, it proves a synergistic nature of good drug interaction; if CDI < 0.7, it indicates a very significant synergistic good drug interaction; if CDI = 1, it indicates an additive nature of good drug interaction; and if CDI > 1, it indicates an antagonistic nature of good drug interaction.

The results showed that the inhibitory effect of each of the aforementioned drugs combined with the sodium ascorbate against the colon cancer cells/melanoma cells was significantly better than that of the corresponding single-drug group, and there was a synergistic effect between the two drugs.

**Example 4 Effect of selective PDE4 inhibitors combined with sodium ascorbate on inhibitory effect of platinum compounds on colon cancer cells/melanoma cells**

The effect of selective PDE4 inhibitors combined with sodium ascorbate on the inhibitory effect of platinum compounds on colon cancer cells/melanoma cells was tested according to the corresponding method in Example 3. The platinum compounds included: oxaliplatin, cisplatin, and carboplatin, respectively; and the selective PDE4 inhibitors included: benzanilide, 4-methylbenzanilide, apremilast, crisaborole, and drotaverine, respectively. The experimental groups were as follows: 1) a single-drug group of selective PDE4 inhibitor, 2) a sodium ascorbate group, 3) a single-drug group of platinum compound, 4) a combinational group of selective PDE4 inhibitor and sodium ascorbate, 5) a combinational group of selective PDE4 inhibitor, sodium ascorbate and platinum compound, in which the concentration of each drug in the combinational group was the same as that in the corresponding single-drug group, and 6) a control group, which was a cell group in which a corresponding solvent having the same volume as that in the single-drug group of selective PDE4 inhibitor was only added.

The results showed that the aforementioned drugs and the sodium ascorbate were capable of significantly strengthening the inhibitory effect of various platinum compounds on tumor cells.

**Example 5 Inhibitory effect of selective PDE4 inhibitors combined with dehydroascorbic acid against colon cancer cells/melanoma cells**

The inhibitory effect of selective PDE4 inhibitors combined with dehydroascorbic acid against colon cancer cells/melanoma cells was tested according to a corresponding method in Example 3. The selective PDE4 inhibitors included: roflumilast, benzamide, 4-methylbenzanilide, apremilast, crisaborole, and drotaverine, respectively. The results showed that the inhibitory effect of each of the aforementioned drugs combined with the dehydroascorbic acid against the colon cancer cells/melanoma cells was significantly better than that of the corresponding single-drug group, and there was a synergistic effect between the two drugs.

**Example 6 Effect of selective PDE4 inhibitors combined with dehydroascorbic acid on inhibitory effect of platinum compounds against colon cancer cells/melanoma cells**

The effect of selective PDE4 inhibitors combined with dehydroascorbic acid on the inhibitory effect of platinum compounds against colon cancer cells/melanoma cells was tested according to the corresponding method in Example 4. The platinum compounds included: oxaliplatin, cisplatin, and carboplatin, respectively; and the selective PDE4 inhibitors included: roflumilast, benzanilide, 4-methylbenzanilide, apremilast, crisaborole, and drotaverineo, respectively. The results showed that the aforementioned drugs and the sodium ascorbate were capable of significantly strengthening the inhibitory effect of various platinum compounds against tumor cells.

**Claims**

1. A pharmaceutical combination, comprising:

   a) a prophylactically and/or therapeutically effective amount of ascorbic acid or a derivative thereof; and
   b) a prophylactically and/or therapeutically effective amount of a selective PDE4 inhibitor.

2. The pharmaceutical combination according to claim 1, further comprising a prophylactically and/or therapeutically effective amount of platinum compound.

3. The pharmaceutical combination according to claim 2, wherein said platinum compound comprises oxaliplatin, cisplatin and/or carboplatin.

4. The pharmaceutical combination according to any one of claims 1 to 3, wherein said selective PDE4 inhibitor comprises apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

5. The pharmaceutical combination according to any one of claims 1 to 3, wherein said selective PDE4 inhibitor comprises a compound represented by Formula I or a pharmaceutically acceptable salt thereof,

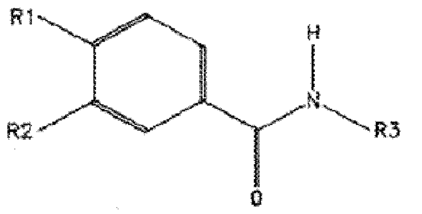

(I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and
R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,
R32 is hydrogen, hydroxy, halogen, amino, trifluoromethyl, $C_1$-$C_4$ alkyl, or $C_1$-$C_4$ alkoxy,
R33 is hydrogen, halogen, $C_1$-$C_4$ alkyl, or $C_1$-$C_4$ alkoxy,
R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl,
R36 is hydrogen or halogen, and
R37 is hydrogen or halogen.

6. The pharmaceutical combination according to any one of claims 1 to 5, wherein said selective PDE4 inhibitor comprises roflumilast or a derivative thereof.

7. The pharmaceutical combination according to claim 6, wherein the derivative of said roflumilast comprises benzanilide and/or 4-methylbenzanilide.

8. The pharmaceutical combination according to any one of claims 1 to 7, wherein the derivative of said ascorbic acid comprises dehydroascorbic acid and/or pharmaceutically acceptable ascorbate.

9. The pharmaceutical combination according to claim 8, wherein said pharmaceutically acceptable ascorbate comprises sodium ascorbate.

10. The pharmaceutical combination according to any one of claims 1-9, wherein an effective amount ratio of said selective PDE4 inhibitor to said ascorbic acid or the derivative thereof is about 1:50 to about 1:500.

11. The pharmaceutical combination according to any one of claims 1-10, wherein a mass ratio of said selective PDE4 inhibitor to said ascorbic acid or the derivative thereof is about 1:50 to about 1:500.

12. The pharmaceutical combination according to any one of claims 2 to 11, wherein an effective amount ratio of said selective PDE4 inhibitor to said ascorbic acid or the derivative thereof to said platinum compound is about 1:50:1 to about 1:500:1.

13. The pharmaceutical combination according to any one of claims 1 to 11, wherein said selective PDE4 inhibitor and said ascorbic acid or the derivative thereof are each present in a different container, respectively.

14. The pharmaceutical combination according to any one of claims 2 to 13, wherein said selective PDE4 inhibitor, said ascorbic acid or the derivative thereof, and said platinum compound are each present in a different container, respectively.

15. The pharmaceutical combination according to any one of claims 1 to 13, wherein said pharmaceutical combination

comprises a first formulation and a second formulation, and said first formulation comprises said ascorbic acid or the derivative thereof and a first pharmaceutically acceptable carrier, and said second formulation comprises said selective PDE4 inhibitor and a second pharmaceutically acceptable carrier.

**16.** The pharmaceutical combination according to any one of claims 2 to 15, wherein said pharmaceutical combination comprises a first formulation, a second formulation, and a third formulation, said first formulation comprises said ascorbic acid or the derivative thereof and a first pharmaceutically acceptable carrier, said second formulation comprises said selective PDE4 inhibitor and a second pharmaceutically acceptable carrier, and said third formulation comprises said platinum compound and a third pharmaceutically acceptable carrier.

**17.** The pharmaceutical combination according to any one of claims 1-13, comprising a pharmaceutical composition, which comprises said selective PDE4 inhibitor and said ascorbic acid or the derivative thereof.

**18.** The pharmaceutical combination according to claim 17, wherein said pharmaceutical composition further comprises a platinum compound.

**19.** The pharmaceutical combination according to claim 17, wherein said selective PDE4 inhibitor in said pharmaceutical composition has a content of about 1% to about 5% (w/w).

**20.** The pharmaceutical combination according to any one of claims 17 to 18, wherein said ascorbic acid or the derivative thereof in said pharmaceutical composition has a content of about 90% to about 98% (w/w).

**21.** The pharmaceutical combination according to any one of claims 18 to 20, wherein said platinum compound in said pharmaceutical composition has a content of about 1% to about 5% (w/w).

**22.** A kit, comprising the pharmaceutical combination according to any one of claims 1 to 21.

**23.** The pharmaceutical combination according to any one of claims 1 to 21 or the kit according to claim 22 for use in the prevention and/or treatment of a tumor.

**24.** The pharmaceutical combination or kit according to claim 23, wherein said tumor comprises a solid tumor and/or a non-solid tumor.

**25.** The pharmaceutical combination or kit according to any one of claims 23 to 24, wherein said tumor comprises a colon cancer and/or melanoma.

**26.** The pharmaceutical combination or kit according to any one of claims 23 to 25, wherein said tumor comprises a tumor or tumor cell having no response to said platinum compound.

**27.** Use of a combination of ascorbic acid or a derivative thereof and a selective PDE4 inhibitor in the preparation of a medicament for preventing and/or treating a tumor.

**28.** Use of a combination of ascorbic acid or a derivative thereof, a selective PDE4 inhibitor, and a platinum compound in the preparation of a medicament for preventing and/or treating a tumor.

**29.** The use according to claim 28, wherein said platinum compound comprises oxaliplatin, cisplatin and/or carboplatin.

**30.** The use according to any one of claims 27 to 29, wherein said selective PDE4 inhibitor comprises apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

**31.** The use according to any one of claims 27 to 29, wherein said selective PDE4 inhibitor comprises a compound represented by Formula I or a pharmaceutically acceptable salt thereof,

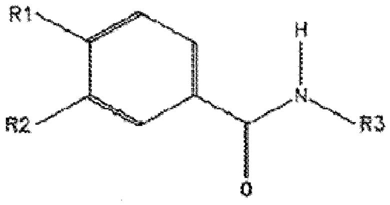

(I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and

R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,

R32 is hydrogen, hydroxy, halogen, amino, trifluoromethyl, $C_1$-$C_4$ alkyl, or $C_1$-$C_4$ alkoxy,

R33 is hydrogen, halogen, $C_1$-$C_4$ alkyl, or $C_1$-$C_4$ alkoxy,

R34 is hydroxy, halogen, cyano, carboxy, $C_1$-$C_4$ alkyl, $C_1$-$C_4$ alkoxy, $C_1$-$C_4$ alkoxycarbonyl, or amino, R35 is hydrogen, halogen, amino, or $C_1$-$C_4$ alkyl,

R36 is hydrogen or halogen, and

R37 is hydrogen or halogen.

**32.** The use according to any one of claims 27 to 31, wherein said selective PDE4 inhibitor comprises roflumilast or a derivative thereof.

**33.** The use according to claim 32, wherein the derivative of said roflumilast comprises benzanilide and/or 4-methylbenzanilide.

**34.** The use according to any one of claims 27 to 33, wherein the derivative of said ascorbic acid comprises dehydroascorbic acid and/or pharmaceutically acceptable ascorbate.

**35.** The use according to claim 34, wherein said pharmaceutically acceptable ascorbate comprises sodium ascorbate.

**36.** The use according to any one of claims 27 to 35, wherein said tumor comprises a solid tumor and/or a non-solid tumor.

**37.** The use according to any one of claims 27 to 36, wherein said tumor comprises a colon cancer and/or melanoma.

**38.** The use according to any one of claims 27 to 37, wherein said tumor comprises a tumor or tumor cell having no response to said platinum compound.

**39.** The use according to any one of claims 27 to 38, wherein said selective PDE4 inhibitor and said ascorbic acid or the derivative thereof are formulated to be simultaneously administered to a subject.

**40.** The use according to any one of claims 27 to 38, wherein said selective PDE4 inhibitor and said ascorbic acid or the derivative thereof are separately formulated to be administered to a subject.

**41.** The use according to any one of claims 28 to 38, wherein said selective PDE4 inhibitor, said ascorbic acid or the derivative thereof, and said platinum compound are formulated to be simultaneously administered to a subject.

**42.** The use according to any one of claims 28 to 38, wherein said selective PDE4 inhibitor, said ascorbic acid or the derivative thereof, and said platinum compound are formulated to be separately administered to a subject.

**43.** The use according to any one of claims 27 to 42, wherein said medicament is formulated such that said selective PDE4 inhibitor is administered at a dose of about 1 mg/kg to about 10 mg/kg.

**44.** The use according to any one of claims 27 to 43, wherein said medicament is formulated such that said ascorbic

acid or the derivative thereof is administered at a dose of about 0.5 g/kg to about 2 g/kg.

**45.** The use according to any one of claims 27 to 44, wherein said medicament is formulated such that said platinum compound is administered at a dose of about 1 mg/kg to about 10 mg/kg.

**46.** The use according to any one of claims 27 to 45, wherein said medicament is formulated such that said selective PDE4 inhibitor and said ascorbic acid or the derivative thereof are administered at a mass ratio of 1:50 to about 1:500.

**47.** The use according to any one of claims 27 to 46, wherein said medicament is formulated such that said selective PDE4 inhibitor and said ascorbic acid or the derivative thereof are administered at an effective amount ratio of about 1:50 to about 1:500.

**48.** The use according to any one of claims 28 to 45, wherein said medicament is formulated such that said selective PDE4 inhibitor, said ascorbic acid or the derivative thereof, and said platinum compound are administered at a mass ratio of about 1:50:1 to about 1:500:1.

**49.** The use according to any one of claims 28 to 48, wherein said medicament is formulated such that said selective PDE4 inhibitor, said ascorbic acid or the derivative thereof, and said platinum compound are administered at an effective amount ratio of about 1:50:1 to about 1:500:1.

**50.** A method for preventing and/or treating a tumor, comprising administering:

a) an effective amount of ascorbic acid or a derivative thereof; and
b) an effective amount of a selective PDE4 inhibitor, to a subject in need thereof.

**51.** The method according to claim 50, further comprising administering an effective amount of platinum compound to a subject in need thereof.

**52.** The method according to claim 51, wherein said platinum compound comprises oxaliplatin, cisplatin and/or carboplatin.

**53.** The method according to any one of claims 50 to 52, wherein said selective PDE4 inhibitor comprises apremilast, crisaborole, drotaverine, CC-1088, BPN14770, GSK356278, HT-0712, TAK-648, zembrin, ASP9831, etazolate, piclamilast, rolipram, cilomilast, GSK256066, AWD 12-281, quinolyl oxazole, DC-TA-46, filaminast and/or glaucine.

**54.** The method according to any one of claims 50 to 52, wherein said selective PDE4 inhibitor comprises a compound represented by Formula I or a pharmaceutically acceptable salt thereof,

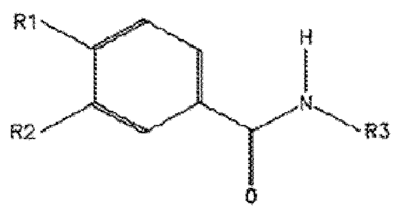

(I)

,

wherein one of R1 and R2 is hydrogen, $C_1$-$C_6$ alkoxy, $C_3$-$C_7$ cycloalkoxy, $C_3$-$C_7$ cycloalkylmethoxy, benzyloxy, or $C_1$-$C_4$ alkoxy fully or partially substituted by fluorine, and the other of R1 and R2 is $C_1$-$C_4$ alkoxy fully or partially substituted by fluorine, and
R3 is phenyl, pyridyl, phenyl substituted by R31, R32 and R33, or pyridyl substituted by R34, R35, R36 and R37, wherein:

R31 is hydroxy, halogen, cyano, carboxy, trifluoromethyl, $C_1$-$C_4$ alkyl, $C_1$-$C_4$ alkoxy, $C_1$-$C_4$ alkoxycarbonyl, $C_1$-$C_4$ alkylcarbonyl, $C_1$-$C_4$ alkylcarbonyloxy, amino, mono- or di-$C_1$-$C_4$ alkylamino, or $C_1$-$C_4$ alkylcarbonylamino,
R32 is hydrogen, hydroxy, halogen, amino, trifluoromethyl, $C_1$-$C_4$ alkyl, or $C_1$-$C_4$ alkoxy,
R33 is hydrogen, halogen, $C_1$-$C_4$ alkyl, or $C_1$-$C_4$ alkoxy,
R34 is hydroxy, halogen, cyano, carboxy, $C_1$-$C_4$ alkyl, $C_1$-$C_4$ alkoxy, $C_1$-$C_4$ alkoxycarbonyl, or amino, R35 is

hydrogen, halogen, amino, or $C_1$-$C_4$ alkyl,
R36 is hydrogen or halogen, and
R37 is hydrogen or halogen.

**55.** The method according to any one of claims 50 to 54, wherein said selective PDE4 inhibitor comprises roflumilast or a derivative thereof.

**56.** The method according to claim 55, wherein the derivative of said roflumilast comprises benzanilide and/or 4-methylbenzanilide.

**57.** The method according to any one of claims 50 to 56, wherein the derivative of said ascorbic acid comprises dehydroascorbic acid and/or pharmaceutically acceptable ascorbate.

**58.** The method according to claim 57, wherein said pharmaceutically acceptable ascorbate comprises sodium ascorbate.

**59.** The method according to any one of claims 50 to 58, wherein said subject is a tumor patient having no response to said platinum compound.

**60.** The method according to any one of claims 50 to 59, wherein said tumor comprises a solid tumor and/or a non-solid tumor.

**61.** The method according to any one of claims 50 to 60, wherein said tumor comprises a colon cancer and/or melanoma.

**62.** The method according to any one of claims 50 to 61, wherein said tumor comprises a tumor or tumor cell having no response to said platinum compound.

**63.** The method according to any one of claims 50 to 62, wherein said selective PDE4 inhibitor and said ascorbic acid or the derivative thereof are simultaneously administered to said subject.

**64.** The method according to any one of claims 50 to 62, wherein said selective PDE4 inhibitor and said ascorbic acid or the derivative thereof are sequentially administered to said subject.

**65.** The method according to any one of claims 51 to 62, wherein said selective PDE4 inhibitor, said ascorbic acid or the derivative thereof, and said platinum compound are simultaneously administered to said subject.

**66.** The method according to any one of claims 51 to 62, wherein said selective PDE4 inhibitor, said ascorbic acid or the derivative thereof, and said platinum compound are sequentially administered to said subject.

**67.** The method according to any one of claims 50 to 66, wherein said selective PDE4 inhibitor is administered at a dose of about 1 mg/kg to about 10 mg/kg.

**68.** The method according to any one of claims 50 to 67, wherein said ascorbic acid or the derivative thereof is administered at a dose of about 0.5 g/kg to about 2 g/kg.

**69.** The method according to any one of claims 51 to 68, wherein said platinum compound is administered at a dose of about 1 mg/kg to about 10 mg/kg.

**70.** The method according to any one of claims 50-69, wherein said selective PDE4 inhibitor and said ascorbic acid or the derivative thereof are administered at a mass ratio of about 1:50 to about 1:500.

**71.** The method according to any one of claims 50-69, wherein said selective PDE4 inhibitor and said ascorbic acid or the derivative thereof are administered at an effective amount ratio of about 1:50 to about 1:500.

**72.** The method according to any one of claims 51 to 69, wherein said selective PDE4 inhibitor, said ascorbic acid or the derivative thereof, and said platinum compound are administered at a mass ratio of about 1:50:1 to about 1:500:1.

**73.** The method according to any one of claims 51 to 69, wherein said selective PDE4 inhibitor, said ascorbic acid or

the derivative thereof, and said platinum compound are administered at an effective amount ratio of about 1:50:1 to about 1:500:1.

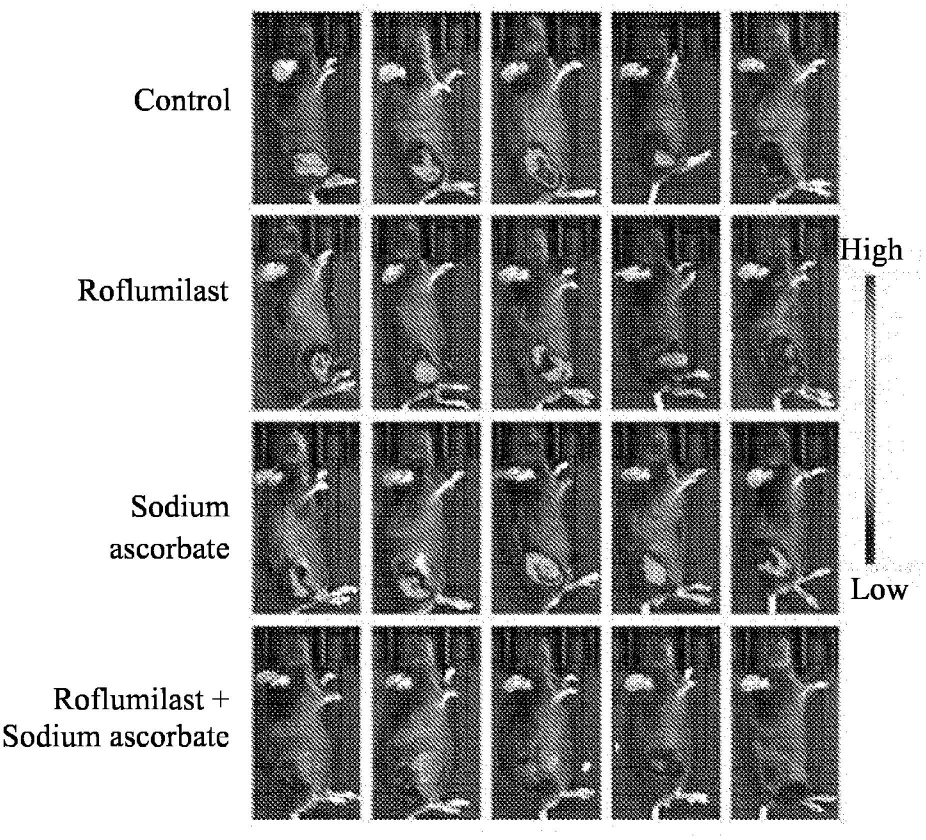
Control
Roflumilast
Sodium ascorbate
Roflumilast + Sodium ascorbate
High
Low

FIG.1

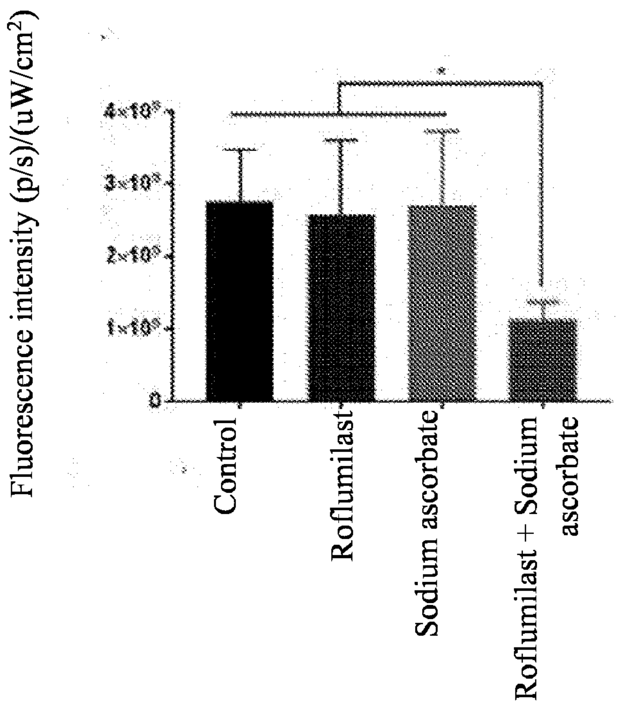
Fluorescence intensity (p/s)/(uW/cm$^2$)
0
*
Control
Roflumilast
Sodium ascorbate
Roflumilast + Sodium ascorbate

FIG.2

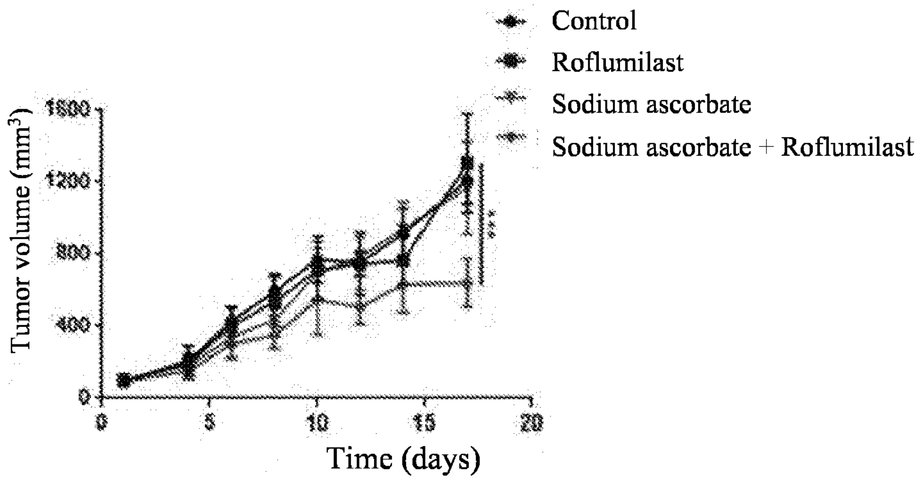


FIG.3

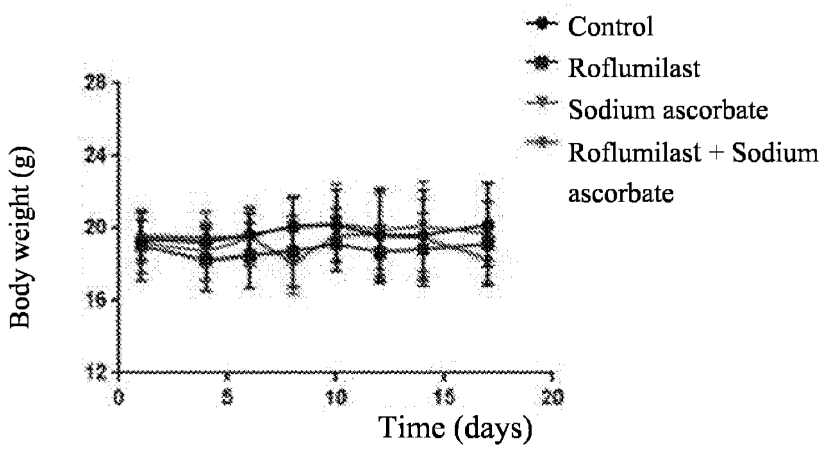


FIG.4

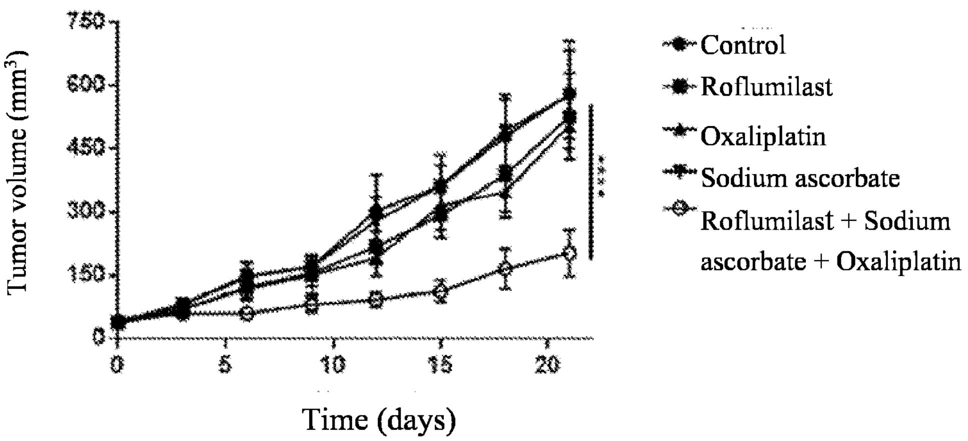


FIG.5

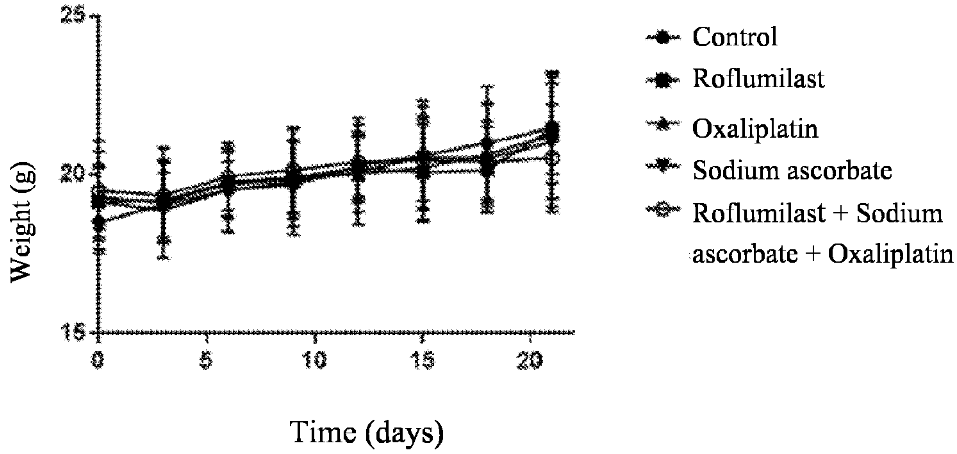
25
20
15
Weight (g)
0
5
10
15
20
Time (days)
Control
Roflumilast
Oxaliplatin
Sodium ascorbate
Roflumilast + Sodium ascorbate + Oxaliplatin

FIG.6

**INTERNATIONAL SEARCH REPORT**

International application No. **PCT/CN2021/088840**

**A. CLASSIFICATION OF SUBJECT MATTER**

A61K 31/375(2006.01)i； A61K 31/355(2006.01)i； A61P 35/00(2006.01)i； A61K 31/44(2006.01)i

According to International Patent Classification (IPC) or to both national classification and IPC

**B. FIELDS SEARCHED**

Minimum documentation searched (classification system followed by classification symbols)

A61K A61P

Documentation searched other than minimum documentation to the extent that such documents are included in the fields searched

Electronic data base consulted during the international search (name of data base and, where practicable, search terms used)

CNPAT, CNKI, WPI, SIPOABS, CAPLUS, REGISTRY, 药物组合物, 抗坏血酸, 维生素C, PDE4, 磷酸二酯酶, 肿瘤, 癌, pharmaceutical composition, vitamine C, tumor, cancer, Apremilast, Crisaborole, Drotaverine, CC-1088, BPN14770, GSK356278, HT-0712, TAK-648, Zembrin, ASP9831, Etazolate, Piclamilast, Rolipram, Cilomilast, GSK256066, AWD 12–281, Quinolyl oxazole, DC-TA-46, Filaminast, Glaucine, Roflumilast

**C. DOCUMENTS CONSIDERED TO BE RELEVANT**

| Category* | Citation of document, with indication, where appropriate, of the relevant passages | Relevant to claim No. |
|---|---|---|
| Y | CN 107007594 A (SUN YAT-SEN UNIVERSITY CANCER CENTER) 04 August 2017 (2017-08-04)<br>claims 1-10 | 1-73 |
| Y | 沈健等 (SHEN, Jian et al.). "磷酸二酯酶4与肿瘤 (Phosphodiesterase-4 and Cancer)" *生命的化学 (Chemistry of Life),* Vol. 35, No. 3, 15 June 2015 (2015-06-15),<br>pp. 350-355 | 1-73 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **09 June 2021** | **14 July 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/088840**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **50-73**
because they relate to subject matter not required to be searched by this Authority, namely:

[1] Claims 50-73 relate to methods of treatment of diseases and therefore do not warrant an international search according to the criteria set out in PCT Rule 39.1(iv), however, a search is made on the use of the prepared drug for said combination.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/088840**

| Patent document cited in search report | Publication date (day/month/year) | Patent family member(s) | Publication date (day/month/year) |
|---|---|---|---|
| CN 107007594 A | 04 August 2017 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5712298 A
- US 5137723 A
- US 5078989 A
- US 20050113312 A1


### Non-patent literature cited in the description


- **NIHRO et al.** *Chem. Pharm. Bull.,* 1991, vol. 39, 1731-1735
- **YAMAMOTO et al.** *J Med. Chem.,* 2002, vol. 45 (2), 462-468